# EUROPEAN PATENT SPECIFICATION

(11) **EP 3 234 189 B1**
(45) Date of publication and mention of the grant of the patent: **12.03.2025**
(21) Application number: 15823586.1
(22) Date of filing: 18.12.2015
(51) Int. Cl.: C12Q 1/6853

(54) **INDEL DETECTION BY AMPLICON ANALYSIS**
INDELDETEKTION MITTELS AMPLICONANALYSE
DÉTECTION D'INDELS PAR ANALYSE D'AMPLICONS

(30) Priority: 19.12.2014 DK 201470809
(43) Date of publication of application: 25.10.2017
(73) Proprietor: Geco ApS, 2800 Kgs. Lyngby (DK)
(72) Inventor: BENNETT, Eric Paul, 2800 Kgs. Lyngby (DK); ZHANG, Yang, 2820 Gentofte (DK)
(74) Representative: AWA Denmark A/S
(86) International application number: PCT/DK2015/050405
(87) International publication number: WO 2016/095931

(56) References cited:
- WO-A1-03/050305
- WO-A2-2006/053259
- DE-A1- 19 934 084
- DE-A1- 19 934 084
- OKA KUMIKO ET AL: "Genotyping of 38 insertion/deletion polymorphisms for human identification using universal fluorescent PCR", MOLECULAR AND CELLULAR PROBES, ACADEMIC PRESS, LONDON, GB, vol. 28, no. 1, 1 February 2014 (2014-02-01), pages 13 - 18, XP009519510, ISSN: 0890-8508, DOI: 10.1016/J.MCP.2013.09.002
- 6 August 2005 (2005-08-06), XP002756525, Retrieved from the Internet <URL:http://www.ebi.ac.uk/ena/data/view/CP000089&display=text> [retrieved on 20050806]

## Description

### Field of invention

The present invention is defined by the appended claims. The present nucleic acids are useful for detecting indels (insertions and deletions) as small as 1 nucleotide in a target nucleic acid. They have a broad applicability for detecting indels following genome editing and can also be used in methods for amplifying a target nucleic acid.

### Background of invention

The emerging gene targeting technologies for precise editing of higher eukaryote genomes such as zinc finger nucleases (ZFNs), transcription activator-like effector nucleases (TALENs), RNA-guided clustered regularly interspaced short palindromic repeats (CRISPRs) or Meganucleases, have revolutionized genome research and enabled studies previously limited to prokaryotes and yeast. These nuclease-based gene editing methods introduce double-stranded DNA breaks and lead to a variety of rearrangements at the breakpoint mediated by cellular repair events including non-homologous end-joining (NHEJ) and homologous recombination. In contrast to the speed by which these editing tools are being optimized and strategies for high throughput use in whole-genome screens are devised, considerably less focus is being devoted to improving capabilities for detection and characterization of the induced indels at the specific breakpoint as well as at potential off-targets. Current approaches available for identification of indels include: i) enzyme mismatch cleavage (EMC) assays, which do not provide sensitive, reliable and accurate identification of the induced indels; and ii) Sanger or next generation DNA sequencing, which is costly, time and labor intensive, and poorly suited for high throughput screening of hundreds or thousands of clones often required to select for desirable multi-allelic editing events that often occur at low frequency. Thus, methods are needed for high-throughput screening of indels.

### Summary of invention

The present nucleic acids are useful for detecting indels (insertions and deletions) as small as 1 nucleotide in a target nucleic acid. They have a broad applicability for detecting indels following genome editing and can also be used in methods for amplifying a target nucleic acid.

### Description of Drawings

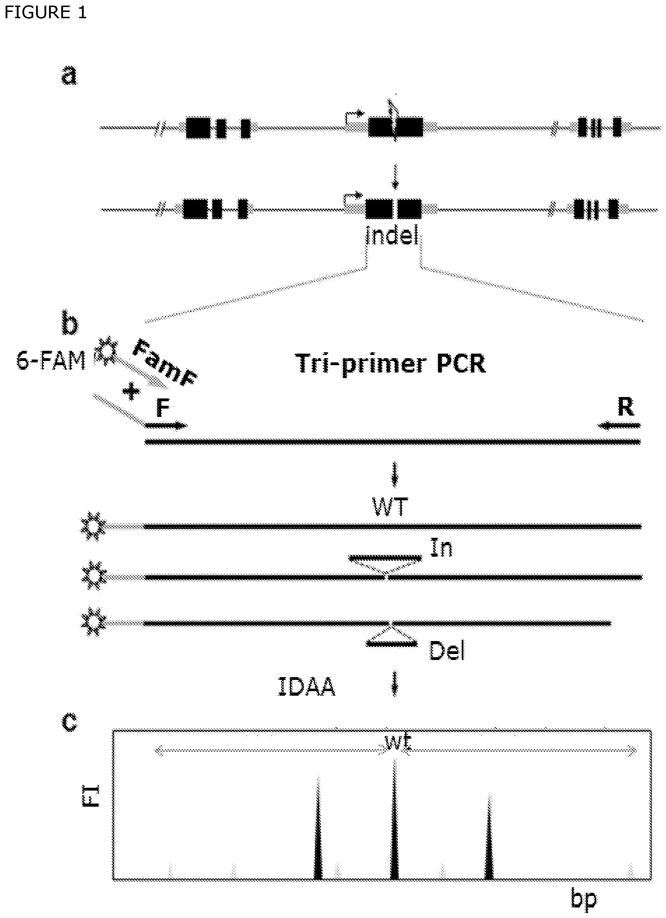

**Fig. 1****. Schematic depiction of the IDAA (Indel Detection by Amplicon Analysis) strategy. (a)** Precise gene targeting creates double-stranded breaks (lightning bolt) at the desired target locus (black box) that through NHEJ introduce indels at the target site. **(b)** Tri-primer PCR of the target region accomplished by use of target specific primers (F/R) flanking the target site and a universal 5'-FAM labelled primer (FamF) specific for a 5'-overhang sequence attached to primer F. Tri-primer PCR results in FAM amplicon labelling. In: insertion; Del: deletion: wt: wild-type. (c) Fluorescently labelled amplicons containing the indels are detected by fragment analysis. Axes represent fluorescence intensity (FI, on Y axis) and amplicon size in base pairs (X axis). The peaks on the left side of the wild-type peak represent deletion events; the peaks on the right side of the wild-type peak represent insertion events.

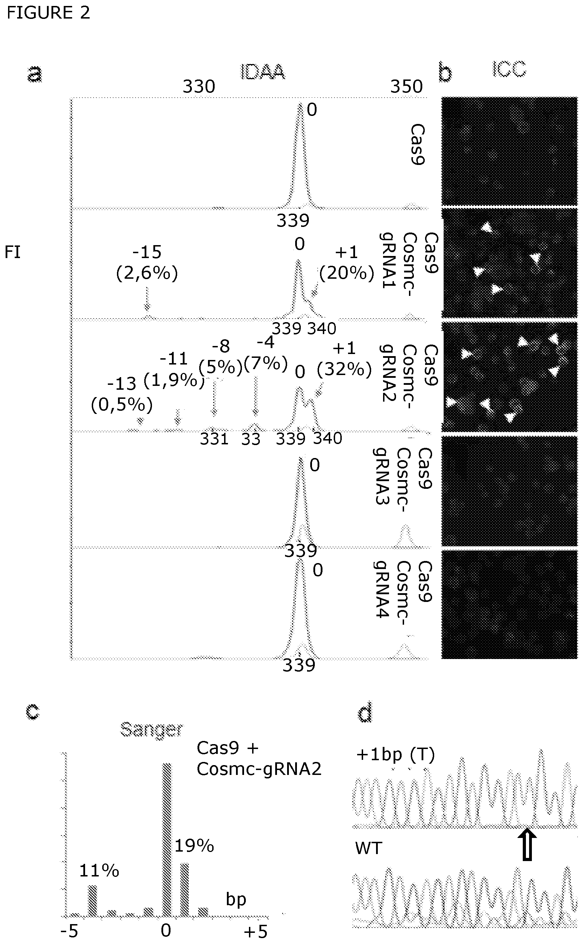

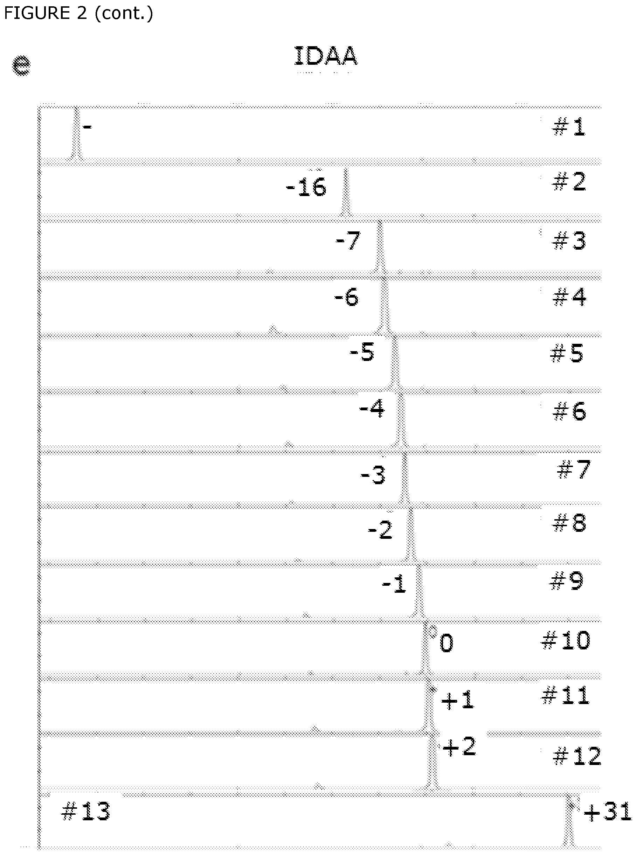

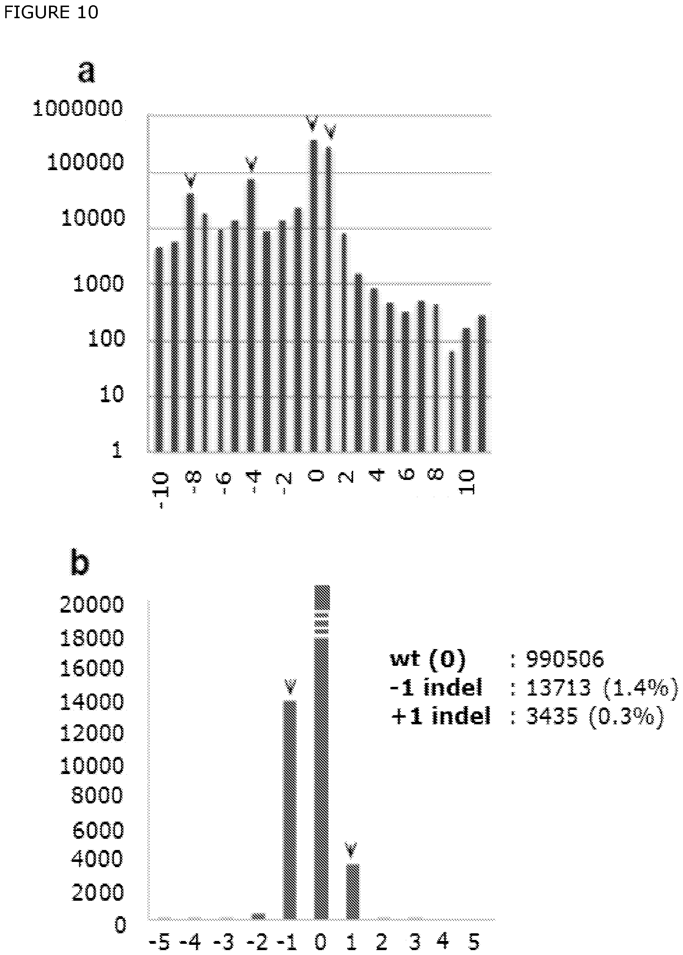

**Fig. 2****. Evaluation of IDAA for detection of indels in gene targeted CHO cell pools and derived single clones. (a)** IDAA of a pool of CHO cells at day two after nucleofection with Cas9 and four different gRNA designs targeting Cosmc. Shown from top are IDAA of cell pools transfected with Cas9 alone, with gRNA1, gRNA2, gRNA3, and gRNA4 (bottom). The position of the unmodified wild type amplicon peak is indicated (0) and amplicon sizes (in bp) as determined by Peak Scanner software are indicated below peaks. Indel sizes determined (in bp) are shown above the most prominent peaks together with cutting efficiencies (calculated from peak area relative to total peak area) in percentage. Total cutting efficiency for gRNA1 and gRNA2 were estimated to 23% and 46%, respectively, while gRNA3 and gRNA4 were inactive. The relative frequency of indels produced by gRNA2 was confirmed by MiSeq deep sequencing (Fig. 10a) which further revealed that the predominant +1 insertion was a thymine insertion three bases upstream of the PAM sequence. The GSLIZ500 standard peaks are shown in light grey. **(b)** Comparative ICC analysis of the corresponding cell pools shown in (a) seven days after nucleofection with a monoclonal antibody (5F4) detecting the de novo induction of truncated O-glycans as a result of complete inactivation of Cosmc18 (arrow heads indicate single positive cells in pool). **(c)** Analysis of indels introduced by Sanger sequencing showing distribution in the -5bp to +5bp range of individual single cell clones. X axis: indel size (bp). Y axis: number of single cell clones (unit: 10). **(d)** Single cell clone Sanger confirmation of the predominant +1bp indel identified by IDAA (arrow). **(e)** Representative IDAA analysis of single cell clones showing the 1bp resolution power of IDAA.

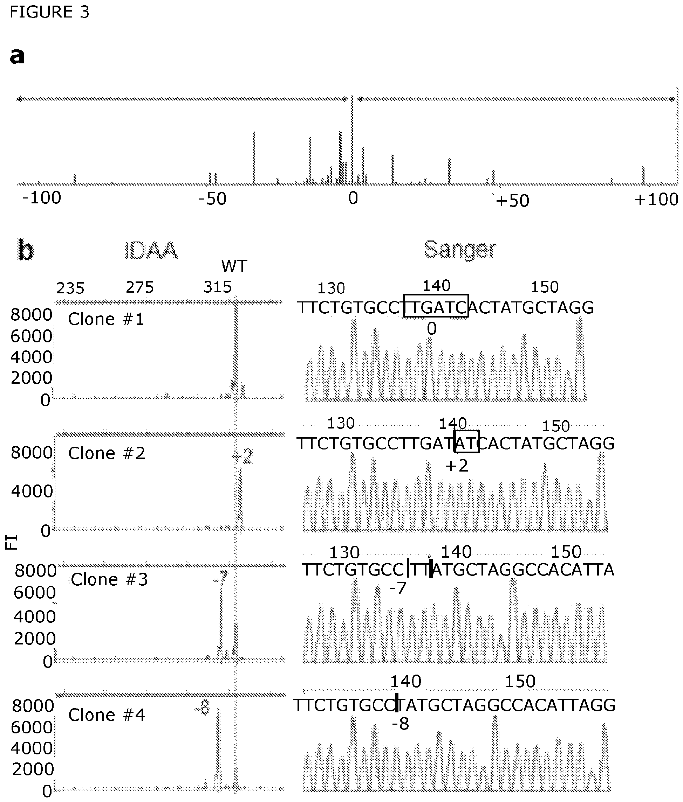

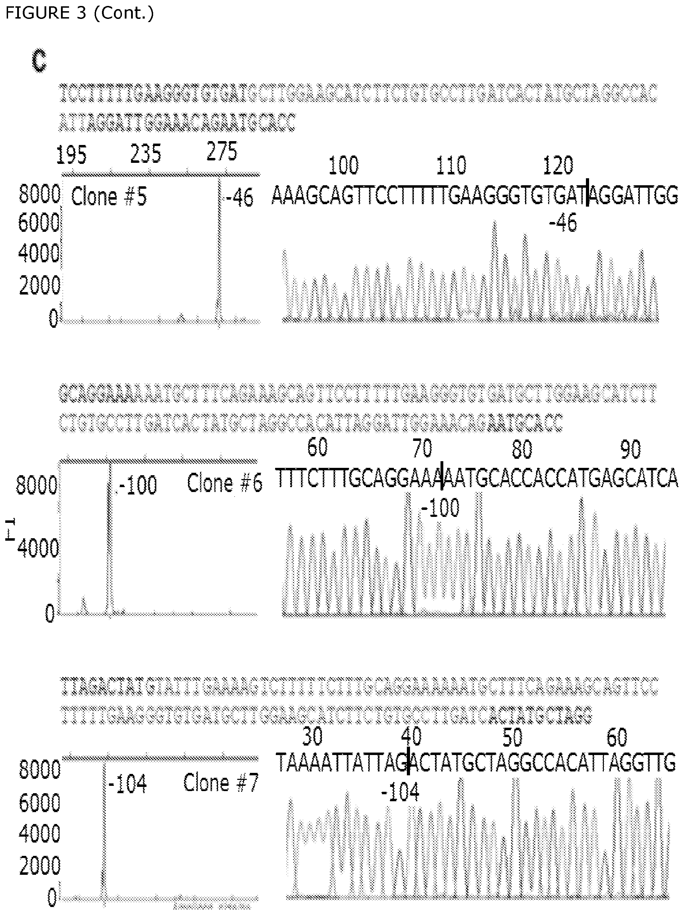

**Fig. 3****. Distribution of indels found in Cosmc-ZFN targeted single cell FACS sorted MC57 clones. (a)** The wild-type allele amplicon is denoted 0 (0 bp indel, highest peak), and 81 clones with indels of varying sizes were identified (deletions on the left, insertions on the right). X axis: indel size (bp). Y axis: number of indels detected. **(b)** Comparative IDAA and Sanger sequencing of representative clones. ZFN cutting site is shown boxed in wt Sanger panel. Indels determined by IDAA are indicated in bp sizes at peaks. For Sanger sequencing the position of the deletion is indicated with a line in the sequence, insertion is boxed (clone #2). **(c)** For the larger deletions detected, the sequence of the targeted region is shown above the IDAA and Sanger panels, with the deleted sequence shown in light grey. For Sanger sequencing the position of the deletion is indicated with a line in the sequence.

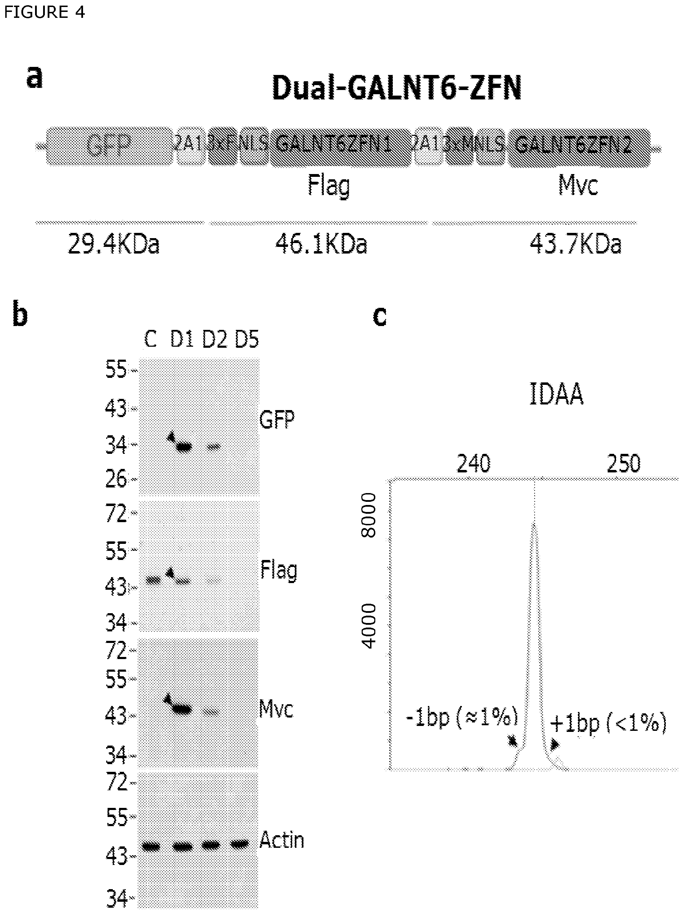

**Fig. 4****. Schematic depiction of the labelled ZFN targeting vector for *GALNT6* (Dual-GALNT6-ZFN). (a)** GFP, 2A peptides (2A1 and 2A2), 3xFLAG (3xF), nuclear localization signal (NLS), ZFN1 and ZFN2 for *GALNT6* (*GALNT6*ZFN1 & 2), and 3xMyc (3xM) are indicated. **(b)** SDS-PAGE Western blot analysis of a pool of K562 cells harvested day 1 (D1), 2 (D2) and 5 (D5) after nucleofection with the Dual-GALNT6-ZFN plasmid targeting vector. Blots were reacted with anti-GFP, anti-FLAG, anti-Myc or anti-actin antibodies as indicated. Arrow heads indicate reactive bands with expected mobilities. For control transfections (C) K562 cells were transfected with monomeric Flag-tagged original ZFNs (Sigma-Aldrich) and harvested day 1. **(c)** IDAA of a HepG2 cell pool at day 2 post nucleofection with Dual-GALNT6-ZFN. The low efficiency and prevalence of +/-1bp indels were confirmed by MiSeq deep sequencing (Fig. 10b). Indel percentages (shown in parenthesis) were calculated from peak areas of the summed indel peaks relative to the total peak area. Y axis: fluorescence intensity. X axis: amplicons size (bp).

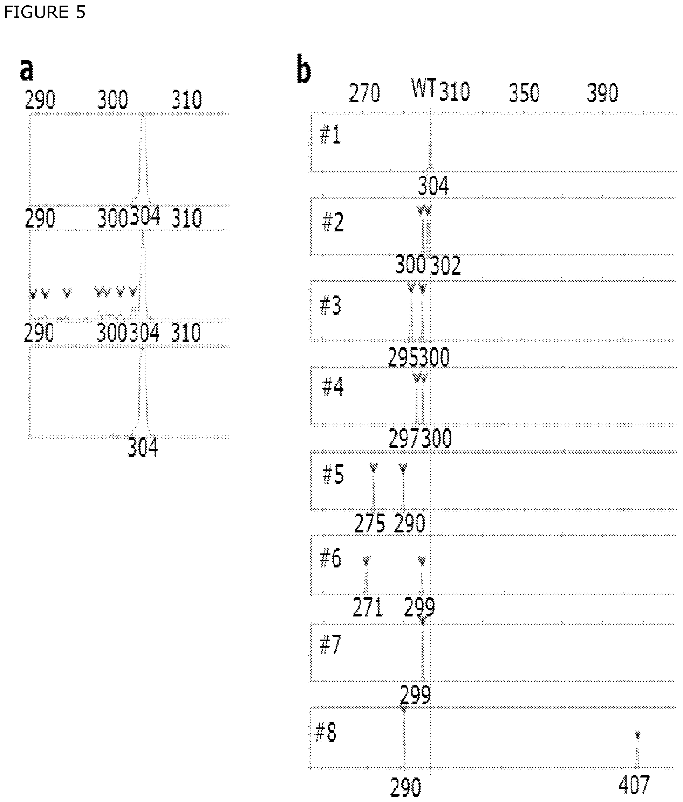

**Fig. 5****. Bi-allelic CHO *St6galnac2* TALEN targeting evaluated by IDAA:** Bi-allelic CHO *St6galnac2* was targeted using a GFP-tagged custom designed TALEN (see Example 1) directed to the target sequence. **(a)** IDAA result of day 2 post transfection unsorted cells (top), FACS bulk sorted cells (middle) or control cells (bottom). Note the increase in indels detected for the bulk-sorted cells (arrow heads) compared to the unsorted cells. Y axis: fluorescence intensity; X axis: amplicons size (bp). **(b)** IDAA results for 8 FACS-sorted, independent single cell clones displaying a variety of different indels. Upper panel represents wild-type (wt) allele, deletions are shown by open arrow heads and insertions by filled arrow heads. A total of 54 single sorted clones were analyzed, 26% (14/54) wt, 26% (14/54) mono allelic targeted and 48% (26/54) bi-allelic targeted. Y axis: fluorescence intensity; X axis: amplicons size (bp).

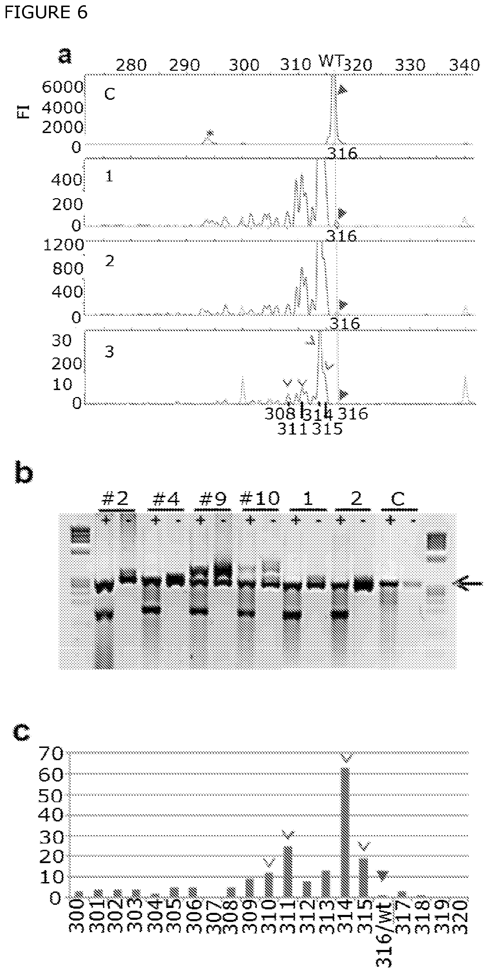

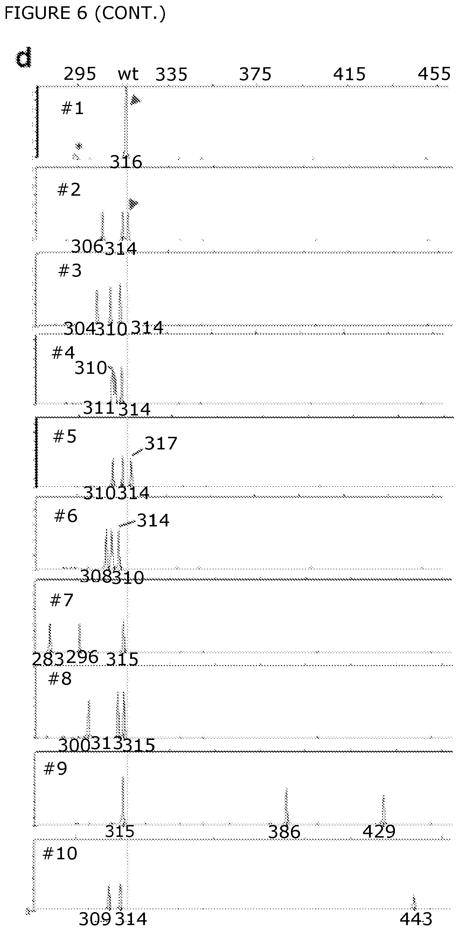

**Fig. 6****. Tri-allelic K562 *KRAS* CRISPR/Cas9 targeting evaluated by IDAA:** Tri-allelic K562 *KRAS* gene was targeted using a plasmid expressing Cas9-2A-GFP and *KRAS* gRNA and indels were detected after 3 consecutive rounds of *KRAS* CRISPR/Cas9 targeting, followed by FACS sorting of GFP positive cells, cell expansion and re-transfection (1st, 2nd and 3rd hit of cells). **(a)** IDAA result of 1^{st} (1), 2nd (2) and 3rd (3) rounds of transfected cells day 2 post transfection. Untransfected cell control (C) is shown in upper panel. The appearance of an unspecific minor peak observed in this assay is indicated by an asterix. The position of the wild type (wt) peak is indicated by filled arrow heads. Notably the wt peak is significantly diminished after the 3rd hit. Dominant peaks are marked by open arrow heads. Y axis: fluorescence intensity; X axis: amplicons size (bp). Amplicon peaks are shown in dark grey and the LIZ500 standard in light grey. **(b)** EMC/T7-assay results of representative 3rd hit single cell sorted clones and 1st and 2nd hit cell pools. Phi-X marker is positioned in the flanking lanes. Arrow indicates the major uncleaved amplicon detected. **(c)** Sanger results from 96 single sorted clones after 3rd hit. All Sanger detected indels from 96 clones are summarized. Note the comparable profile for the indels detected in the pool of cells shown in panel A marked by open arrow heads. Y axis: number of alleles detected; X axis: amplicons size (bp). **(d)** Representative IDAA results from 3rd hit single cell clones displayed in panel b. Notably only one wt allel was detected.

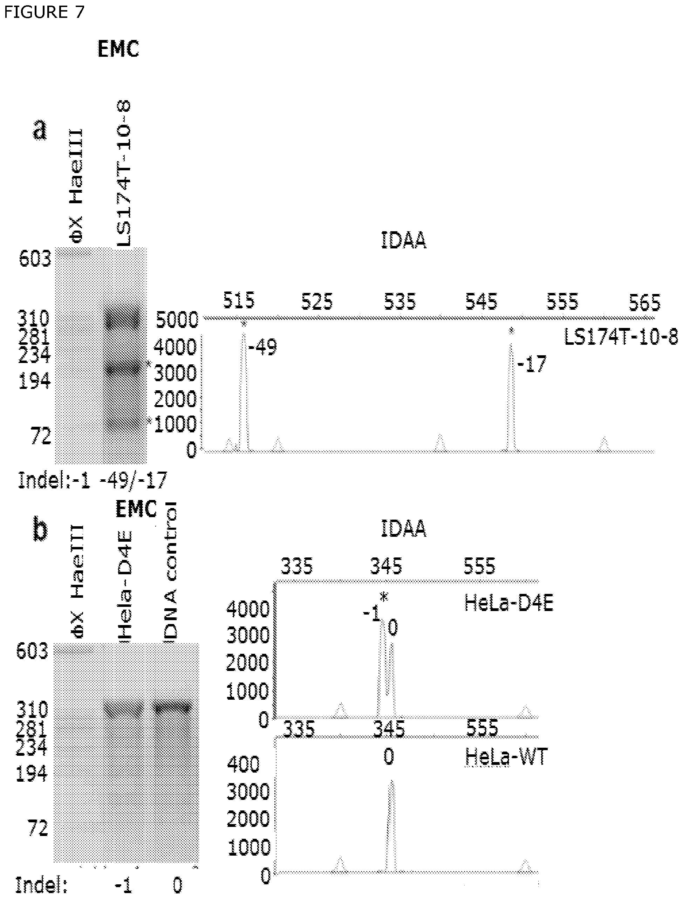

**Fig. 7****. Comparative EMC and IDAA analysis of ZFN targeted clones with large indels or single base indel.** EMC assays are commonly based on T4 endonuclease VII (T4E7), endonuclease V (EndoV), T7 endonuclease I (T7EI), CELI or Surveyor nuclease. **(a)** EMC (T7EI) assay of amplicons derived from a single LS174T clone (#10-8) targeted with Dual-GALNT6-ZFN. Cleaved products are indicated with asterix. Comparative IDAA of the same clone shown to the right. **(b)** EMC (T7EI) assay of amplicons derived from a single HeLa clone (DE4) targeted with COSMC-ZFN. Comparative IDAA of the same clone demonstrating a monoallelic -1bp deletion (indicated with and asterix) shown to the left, relative to the intact HeLa WT peak(0). Unmarked minor light grey peaks represent the GSLIZ600 standard.

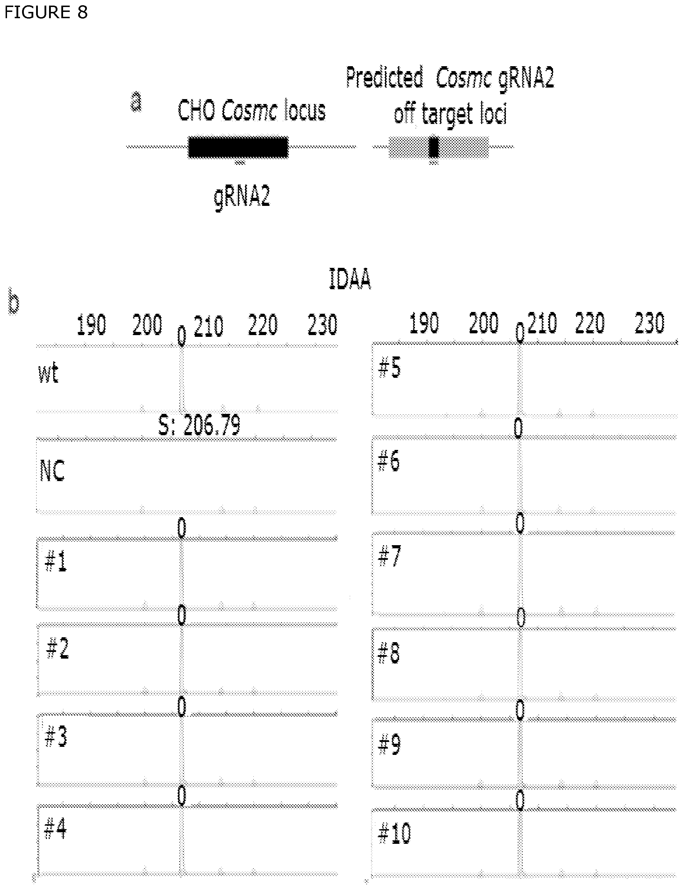

**Fig. 8. (a)** Schematic depiction of the gRNA2 target region in the *Cosmc* gene locus and candidate off-target loci with 1-4 mismatches. 1 mismatch: 0; 2 mismatches: 1; 3 mismatches: 10; 4 mismatches: 178. **(b)** IDAA of the top off-target candidate region with 2 mismatches in 10 single CHO cell clones. The Cosmc Cas9/gRNA2 targeted single cell sorted clones were from the experiment presented in Figure 2. The position of the intact amplicon is indicated by 0 above peak (0bp indel). No off-target events were detected and this was confirmed by Sanger sequencing. LIZ600 marker positions are shown as unmarked peaks within diagrams. NC: negative control. Y axis: fluorescence intensity; X axis: amplicons size (bp).

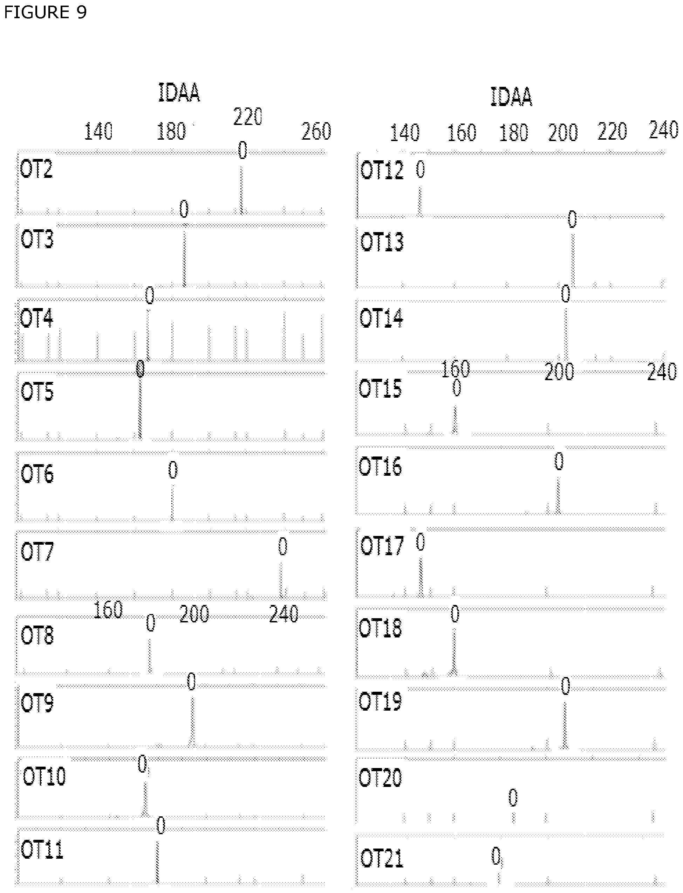

**Fig. 9****. IDAA of the top 21 off-target (OT) loci** are shown for a single CHO clone (#7) targeted with *Cosmc* Cas9-gRNA2 from the experiment described for Figure 2. IDAA of all 21 off-targets was performed on additional 9 CHO clones, and no off-target events were identified. IDAA OT1 results are shown in Fig. 8. Position of the only detected intact amplicon (representing the unmodified off target) is indicated by 0 above peak (0 bp indel). Results were verified by Sanger sequencing of all amplicons analyzed. LIZ600 marker positions are shown as unmarked peaks within diagrams. Note, that the relative differences in amplicon product yields for the different targets shown, give rise to considerable variation in the intensities of the LIZ600 marker shown as unmarked peaks in the respective chromatograms.
**Fig. 10****. Number of indels detected in cell pools. (a)** CHO cell pool, day 2 post CRISPR/Cas9 *Cosmc* gRNA2 transfection. Number of indels detected with indel sizes ranging from -10 bp to +10 bp are shown. Note the profiles for the -4bp, 0, +1 bp match the IDAA profiles shown in Figure 2a. Y-axis (number of indels) is logarithmic. **(b)** Human HepG2 cell pool, day 2 post Dual-GALNT6-ZFN transfection. Number of indels detected with indel sizes ranging from -5 bp to +5 bp are shown. Note the profile and indel frequencies for the -1bp, 0, +1 bp match the IDAA profiles shown in Figure 4c.

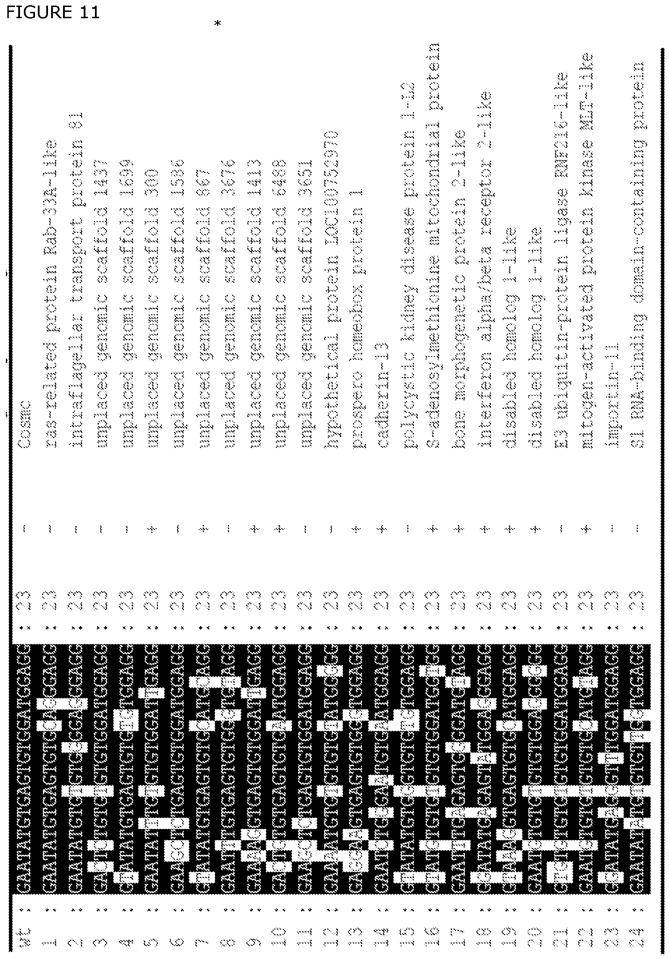

**Fig. 11****. "Off target" mismatch distribution at top 24 sites.** "wt": wild type; + and - indicate the strand. The gRNA target is indicated to the right. The asterisk indicates the last base in the gRNA preceding the PAM seed sequence.

### Definitions

Practice of the methods, as well as preparations and use of the compositions disclosed herein employ, unless otherwise indicated, conventional techniques in molecular biology, biochemistry, bioinformatics, cell culture, recombinant DNA and related fields as are known in the art.

Adaptamer: the term herein refers to part of a nucleic acid sequence such as a primer where the adaptamer part does not hybridize to the target nucleic acid but instead is identical to the sequence of another nucleic acid such as another primer. The adaptamer is preferably comprised at the 5'-end of the primer.

Amplification reaction: an amplification reaction as understood herein is any reaction during which a target nucleic acid such as a genomic region of interest, a gene of interest, a locus of interest on a plasmid, is amplified. Polymerase chain reaction (PCR) is an example of such a reaction.

Amplicon: the term "amplicon" refers herein to a nucleic acid sequence obtained after amplification of a target nucleic acid, e.g. by PCR.

Annealing: annealing as used herein refers to the process by which complementary sequences of single-stranded nucleic acids or nucleic acid analogues pair by hydrogen bond formation, resulting in a double-stranded molecule.

Base pair (bp): a base pair shall herein refer to two complementary nucleotides linked by hydrogen bonds. The term is also used as a length measurement, interchangeably with "nt" (nucleotide).

Complementarity: as used herein, the terms "complementarity" or "complementary" are used in reference to polynucleotides (i.e., a sequence of nucleotides) related by the base-pairing rules. Complementarity may be "partial," in which only some of the nucleic acids' bases are matched according to the base pairing rules, or there may be "complete" or "total" complementarity between the nucleic acids. The degree of complementarity between nucleic acid strands has significant effects on the efficiency and strength of their hybridisation to one another.

Cutting efficiency: this term refers to the relative efficiency by which a gene targeting tool such as CRISPR/Cas9, ZFN, TALEN or other is able to induce indels at a specific locus in the genome of any given species or cell. Indels are generated after introduction of a double-strand break (DSB), i.e. induced by precise gene targeting. Subsequent to this, the double strand break is being repaired through either homologous recombination (HR) or non-homologous end joining (NHEJ). In the absence of a homologous template, non-homologous end-joining (NHEJ) is the predominant repair pathway. NHEJ repairs DSBs by joining the two ends together and usually produces no mutations, provided that the cut is clean and uncomplicated, but in some instances the repair will be imperfect, resulting in an insertion or deletion of basepairs, producing frame-shift mutations and preventing the production of the protein of interest. The term cutting thus refers to a gene targeting tool's ability to induce double stranded breaks and indels at a predetermined site in the genome.

Denaturation: denaturation" or "melting" refers to the process by which double-stranded nucleic acid or nucleic acid analogue molecules unwind and separate into single-stranded strands through the breaking of hydrogen bonding between the bases.

Detection: the term "detection" as used herein refers to the quantitative or qualitative identification of DNA species such as, but not limited to, diffentially sized amplicons within a sample. The term "detection assay" as used herein refers to a kit, test, or method performed for the purpose of detecting an analyte nucleic acid within a sample. Detection assays produce a detectable signal or effect when performed in the presence of the target analyte, and include but are not limited to assays incorporating the processes of hybridization, nuclei acid amplification, nucleotide sequencing or primer extension. A detection assay configured for target detection is a collection of assay components that together are capable of producing a detectable signal when the target nucleic acid is present.

Downstream: as used herein, the term "downstream" applies to the end region of a nucleic acid, or to a region downstream of the nucleic acid. The term "downstream region" thus may refer to a region comprised within the target nucleic acid or the nucleic acid of interest, or to a region outside the region of interest.

Elongation primer: an elongation primer is a nucleic acid molecule capable of hybridizing or annealing to a target nucleic acid and of priming a PCR reaction.

Fluorophore/Fluorescent moiety: a fluorescent moiety or fluorophore as understood herein is any substance that can re-emit light upon excitation. Fluorescence is generated when the fluorophore, lying in its ground state, absorbs light energy at a short wavelength, creating an excited electronic singlet state, and emits light energy at a longer wavelength, creating a relaxed singlet state. The fluorophore then returns to its ground state.

Hybridisation: as used herein, the term "hybridisation" or "hybridise" is used in reference to the non-covalent, sequence-specific interaction between two complementary strands of nucleic acids into a single complex. Hybridisation and the strength of hybridisation (i.e., the strength of the association between the nucleic acids) are influenced by such factors as the degree of complementarity between the nucleic acids, the stringency of the conditions involved, the melting temperature (Tm) of the formed hybrid, and the G:C ratio within the nucleic acids.

Indel: the term "indel" stands for "insertion/deletion" and refers herein to insertion or deletion events in a nucleic acid molecule. While insertion and deletion events may occur at the same time in the same nucleic acid e.g. during gene targeting, an indel results in a net change in the number of nucleotides, typically between 1 and 50. Indels often result in frameshift mutations, except when the number of inserted/deleted nucleotides is a multiple of 3.

Melting temperature (Tm): the "Tm" or "melting temperature" or "melting point", of an oligonucleotide refers to the temperature (in degrees Celsius) at which 50% of the molecules in a population of a single-stranded oligonucleotide are hybridised to their complementary sequence and 50% of the molecules in the population are not hybridised to their complementary sequence. The Tm can be determined empirically by means of a melting curve or it can be calculated using software well known in the art. Nucleic acid: the term refers to a multimeric compound comprising two or more covalently bonded nucleosides or nucleoside analogues having nitrogenous heterocyclic bases, or base analogues, where the nucleosides are linked together by phosphodiester bonds or other linkages to form a polynucleotide. Nucleic acids include RNA, DNA, or chimeric DNA-RNA polymers or oligonucleotides, and analogues thereof, including peptide nucleic acid (PNA), Morpholino and locked nucleic acid (LNA), as well as glycol nucleic acid (GNA) and threose nucleic acid (TNA). Any nucleic acid analogue which will be recognised by a person skilled in the art to possess properties such that it can be used for the molecular beacons described herein can be used for embodiments of the present invention. Sugar moieties of the nucleic acid may be either ribose or deoxyribose, or similar compounds having known substitutions such as, for example, 2'-methoxy substitutions and 2'-halide substitutions (e.g., 2'-F). Nitrogenous bases may be conventional bases (A, G, C, T, U), analogues thereof (e.g., inosine, 5-methylisocytosine, isoguanine), which include derivatives of purine or pyrimidine bases (e.g., N4-methyl deoxygaunosine, deaza- or aza-purines, deaza- or aza-pyrimidines, pyrimidine bases having substituent groups at the 5 or 6 position, purine bases having an altered or replacement substituent at the 2, 6 and/or 8 position, such as 2-amino-6-methylaminopurine, 06-methylguanine, 4-thio-pyrimidines, 4-amino-pyrimidines, 4-dimethylhydrazine-pyrimidines, and 04-alkyl-pyrimidines, and pyrazolo-compounds, such as unsubstituted or 3 -substituted pyrazolo[3,4-d]pyrimidine). Nucleic acids may include "abasic" residues in which the backbone does not include a nitrogenous base for one or more residues. A nucleic acid may comprise only conventional sugars, bases, and linkages as found in RNA and DNA, or may include conventional components and substitutions (e.g., conventional bases linked by a 2'-methoxy backbone, or a nucleic acid including a mixture of conventional bases and one or more base analogues). Nucleic acids may comprise "locked nucleic acids" (LNA), in which one or more nucleotide monomers have a bicyclic furanose unit locked in an RNA mimicking sugar conformation, which enhances hybridisation affinity toward complementary sequences in single-stranded RNA (ssRNA), single-stranded DNA (ssDNA), or double-stranded DNA (dsDNA). Nucleic acids may comprise modified bases to alter the function or behaviour of the nucleic acid, e.g., addition of a 3'-terminal dideoxynucleotide to block additional nucleotides from being added to the nucleic acid. Synthetic methods for making nucleic acids in vitro are well known in the art although nucleic acids may be purified from natural sources using routine techniques.

Nucleotide: a "nucleotide" (or "nt") as understood herein is a subunit of a nucleic acid consisting of a phosphate group, a 5-carbon sugar and a nitrogenous base. The 5-carbon sugar found in RNA is ribose. In DNA, the 5-carbon sugar is 2'-deoxyribose. The term also includes analogues of such subunits. In the present context, the unit "nt" or the unit "bp" (basepairs) will be used interchangeably to designate the length of an indel.

Polymerase Chain Reaction (PCR): the polymerase chain reaction (PCR) is a molecular biology technique allowing amplification of a single or a few copies of a nucleic acid across several orders of magnitude, generating thousands to millions of copies of a particular nucleic acid sequence. A PCR typically requires: a nucleic acid template that contains the nucleic acid region (target) to be amplified; at least two primers that are complementary to the 3'-ends of each of the sense and anti-sense strand of or surrounding the target; a polymerase; deoxynucleoside triphosphates (dNTPs; nucleotides containing triphosphate groups); a buffer solution, providing a suitable chemical environment for optimum activity and stability of the polymerase; divalent cations, magnesium or manganese ions; monovalent cation potassium ions.

Three temperatures are important and characteristic of a PCR:
i) the denaturation temperature is the temperature at which the nucleic acid present in the reaction gets denatured, i.e. shifts from being double-stranded to single-stranded. During denaturation, the sample nucleic acid becomes denatured and primers that can be hybridised to the target are released as single-stranded molecules. Thus all nucleic acids are accessible to the polymerase and complementary nucleic acid sequences can hybridise once the temperature is shifted to the annealing temperature. The denaturation temperature is typically within the range of 90°C to 100°C. The denaturation temperature should be greater than the melting temperature of the hybridised primer-target complex. The duration of the denaturation step is determined by the user and depends on the nature of the target and of the primers used. Typically, denaturation lasts between 2 seconds and 3 minutes.
ii) the annealing temperature is the temperature at which the primers can hybridise to their complementary sequences on the target; the annealing temperature may be in the range of 43°C to 70°C. For most applications, the annealing temperature is typically in the range of 52°C to 65°C. The annealing temperature should be lower than the melting temperature of the hybridised primer-target complex. The duration of the annealing step is determined by the user and depends on the nature of the target and of the primers used. Typically, annealing lasts between 2 seconds and 3 minutes.
iii) the elongation temperature is the temperature at which the polymerase is active and can elongate the target; synthesis is primed when the primers are hybridised to their complementary sequence. The elongation temperature can vary depending on the nature of the polymerase. The most common elongation temperatures are 68°C and 72°C, but any temperature in the range of 65°C to 75°C can be considered. The duration of the elongation step is determined by the user and depends on the nature of the target, of the polymerase and of the primers used; more particularly it depends on the length of the target molecule to be amplified and on the synthesis speed of the polymerase used.

The three cycles i), ii) and iii) are typically repeated 20 to 35 times, and may be followed by a final elongation step, often performed at the elongation temperature, to ensure full extension of any remaining single-stranded nucleic acids. In so-called 2-step PCR ii) and iii) are combined at the same temperature. The PCR can be performed in liquid phase in a thermocycler or in solid phase. For solid-phase PCR a microfluidic device may be used, in which different primer pairs can be immobilised in known positions on the surface of the device, while the fluid running through the device (also termed "reaction mixture") contains all the other reagents necessary for the PCR, such as polymerase, template DNA, dNTPs, buffer, and primers. Alternatively, only one primer is immobilised on the device, while the other is comprised in the reaction solution.

Primer: a primer is a nucleic acid which is able to at least partly bind to a target nucleic acid. Primers are designed so that their sequence is complementary to a portion of the target nucleic acid to be amplified during an amplification reaction. In a typical PCR, two primers are used, each hybridizing on one side of the region to amplify (one primer upstream and one downstream). Primers comprise at least a sequence complementary to the target, but may also comprise additional nucleic acid sequences such as an adaptamer tail or appended extension. A primer is an oligonucleotide, which is capable of acting as a point of initiation of synthesis when placed under conditions in which production of an elongation which is complementary to a target nucleic acid strand is induced (e.g. in a PCR reaction). Primers thus allow an enzyme such as a DNA polymerase to copy the nucleic acid template (the target nucleic acid). Primers can be modified in several ways, such as labelling by e.g. a fluorophore. In order for primers to allow amplification by a polymerase, it is not necessary that the complementary sequence matches the portion of the target nucleic acid exactly; a proportion of mismatches does not prevent the reaction from occurring, if the primer is able to bind at least partly to the target sequence, as is well known in the art. Primers are sufficiently long to specifically prime the synthesis of extension products and are single stranded oligo-nucleotide sequence of usually 15-30 nucleotides or more in length and complementary in sequence to a polynucleotide target sequence, e.g. a locus contained in genomic DNA.

Target nucleic acid: the term "target nucleic acid" or "target", when used in reference to nucleic acid detection or analysis method, refers to a nucleic acid having a particular sequence of nucleotides to be detected or analysed, e.g. in a sample suspected of containing the target nucleic acid. When used in reference to the polymerase chain reaction, "target" generally refers to the region of nucleic acid bounded by primers used in the reaction. Thus, the target is to be sorted out from other nucleic acid sequences that may be present in a sample.

Targetant: a targetant as understood herein refers to a cell or a cell population in which gene targeting events may have taken place. In the context of genome editing, a targetant is a transformant or transfectant which may be positive (successful gene targeting) or negative (unsuccessful gene targeting).

Tri-primer PCR: a tri-primer PCR is a PCR performed with three primers, where two are elongation primers typically used for PCR (a forward primer and a reverse primer each annealing to a region of the target nucleic acid to be amplified) and a universal primer. In the present context, at least one of the elongation primers comprises a tail which does not anneal to the target nucleic acid but instead functions as an adaptamer allowing a third primer to anneal to an amplicon having integrated the adaptamer sequence after at least one amplification event. The third primer can be a universal primer.

Upstream: as used herein, the term "upstream" applies to the end region of a nucleic acid, or to a region upstream of the nucleic acid. The term "upstream region" thus may refer to a region comprised within the target nucleic acid or the nucleic acid of interest, or to a region outside the region of interest.

Universal primer: a universal primer is a primer designed to anneal to a sequence complementary to the adaptamer sequence comprised within an amplicon generated using an elongation primer comprising an adaptamer. The universal primer will thus be able to hybridize to the complementary sequence of the tailed extension primer. The universal primer requires prior incorporation of the extension primer into amplicons, before its ability to prime extension and amplicon incorporation. Universal primers are sometimes labelled, e.g. by a fluorophore, either internally or externally (i.e. in the 3'- or 5'-end). Typically, when a 5'-to-3' DNA polymerase is used, the universal primer is labelled in the 5'-end.

Wild type: The term "wild type" refers to a gene or a gene product that has the characteristics of that gene or gene product when isolated from a naturally occurring source. A wild-type gene is that which is most frequently observed in a population and thus is arbitrarily designed the normal or wild-type form of the gene. In contrast, the terms "modified", "mutant" or "variant" refer to a gene or gene product that displays modifications in sequence and/or functional properties (i.e., altered abrogated function due to the introduction of indels following gene editing) when compared to the wild-type gene or gene product.

### Detailed description of the invention

The present invention is based on the finding that the nucleic acids disclosed herein allow detection of a broad range of indels down to the single base level. The method disclosed herein is suitable for high-throughput screening based on indel detection by amplicon analysis, e.g. detection of indels induced by cellular precise gene targeting. A tri-primer amplification method for detecting genetic variations is described in DE19934084 A1.

The present invention is as defined in the claims.

The invention employs a nucleic acid comprising the sequence 5'-NNNTGACCGGCAGCAAAATTG-3' (SEQ ID NO: 1) or a variant thereof having at least 85% identity to SEQ ID NO: 5, the 5'-end of said nucleic acid or variant thereof being labelled with a fluorophore.

Which is used in a method for detecting an indel in a target nucleic acid, said method comprising the steps of:
a) performing a triprimer amplification reaction to amplify the target nucleic acid, said reaction comprising the steps of:
   i. providing a first elongation primer capable of annealing to a region of a target nucleic acid;
   ii. providing a second elongation primer capable of annealing to another region of said target nucleic acid;
      wherein at least one of the first and the second primers comprises an adaptamer sequence in its 5'-end;
   iii. providing a universal primer, said universal primer being labelled by a fluorophore in its 5'-end, and said universal primer being identical to the adaptamer sequence;
      thereby obtaining fluorophore-labelled amplicons;
b) analysing the size of said fluorophore-labelled amplicons in order to determine whether an indel is present.

In yet another aspect, not being part of the present invention, a method for synthesizing a nucleic acid is described herein.

Outside the scope of the present invention, yet another aspect, the use of a nucleic acid as described herein in a method of detecting an indel in a target nucleic acid.

Outside the scope of the present invention, yet another aspect, the invention relates to the use of a nucleic acid as described herein in a method of amplification of a target nucleic acid.

Outside the scope of the present invention, yet another aspect, a kit comprising a nucleic acid is described herein and instructions for use.

### Nucleic acid

Herein is provided a nucleic acid comprising the sequence 5'-NNNTGACCGGCAGCAAAATTG-3' (SEQ ID NO: 1) or a variant thereof comprising a sequence having at least 85% identity to SEQ ID NO: 5, the 5'-end of said nucleic acid or variant thereof being labelled with a fluorophore. N is any nucleotide base such as a guanine base, an adenine base, a thymine base or a cytosine base. In some embodiments, the nucleic acid consists of 5'-NNNTGACCGGCAGCAAAATTG-3' (SEQ ID NO: 1) or a variant thereof comprising a sequence having at least 85% identity to SEQ ID NO: 5. Thus the nucleic acid may comprise or consist of any of SEQ ID NO: 3 or SEQ ID NOs: 6 to 68.

In a preferred embodiment, the nucleic acid is 5'-AGCTGACCGGCAGCAAAATTG-3' (SEQ ID NO: 3).

In some embodiments, the nucleic acid is a variant of 5'-NNNTGACCGGCAGCAAAATTG-3' (SEQ ID NO: 1) having at least 85% identity to 5'-TGACCGGCAGCAAAATTG-3' (SEQ ID NO: 5). Thus in one embodiment, the variant comprises a sequence having at least 85% identity, such as at least 86% identity, such as at least 87% identity, such as at least 88% identity, such as at least 89% identity, such as at least 90% identity, such as at least 91% identity, such as at least 92% identity, such as at least 93% identity, such as at least 94% identity, such as at least 95% identity, such as at least 96% identity, such as at least 97% identity, such as at least 98% identity, such as at least 99% identity, such as 100% identity, to SEQ ID NO: 5.

Thus in some embodiments, the nucleic acid is a variant of SEQ ID NO: 1 having between 0 and 3 mutations compared to SEQ ID NO: 5, such as 0 mutation, such as 1 mutation, such as 2 mutations, such as 3 mutations. Any nucleotide of SEQ ID NO: 5 can be mutated to any of a guanine base, an adenine base, a thymine base or a cytosine base.

The nucleic acid may further comprise an additional nucleotide base in the 5'-end. In particular, the additional base may be a guanine base, an adenine base, a thymine base or a cytosine base. In one embodiment, the additional base is a guanine base and the nucleic acid or variant thereof comprises or consists of the sequence 5'-GNNNTGACCGGCAGCAAAATTG-3' (SEQ ID NO: 2). N is any nucleotide base such as a guanine base, an adenine base, a thymine base or a cytosine base. Thus the nucleic acid may comprise or consist of any of SEQ ID NO: 4 or SEQ ID NOs: 69 to 131.

In one embodiment, the nucleic acid comprises 5'-GAGCTGACCGGCAGCAAAATTG-3' (SEQ ID NO: 4). In another embodiment, the nucleic acid is 5'-GAGCTGACCGGCAGCAAAATTG-3' (SEQ ID NO: 4).

In some embodiments, the nucleic acid is a variant of 5'-GNNNTGACCGGCAGCAAAATTG-3' (SEQ ID NO: 2) having at least 85% identity to 5'-TGACCGGCAGCAAAATTG-3' (SEQ ID NO: 5). Thus in one embodiment, the variant comprises a sequence having at least 85% identity, such as at least 86% identity, such as at least 87% identity, such as at least 88% identity, such as at least 89% identity, such as at least 90% identity, such as at least 91% identity, such as at least 92% identity, such as at least 93% identity, such as at least 94% identity, such as at least 95% identity, such as at least 96% identity, such as at least 97% identity, such as at least 98% identity, such as at least 99% identity, such as 100% identity to SEQ ID NO: 5.

### Fluorophore

The nucleic acid described herein is labelled in its 5'-end with a fluorophore. Suitable fluorophores are known to the skilled person and include 6-carboxyfluorescein (6-FAM), Alexa Fluor^{®} 350, DY-415, ATTO 425, ATTO 465, Bodipy^{®} FL, Alexa Fluor^{®} 488, fluorescein isothiocyanate, ATTO 488, Oregon Green^{®} 488, Oregon Green^{®} 514, Rhodamine Green^{™}, 5'-Tetrachloro-Fluorescein, ATTO 520, 6-carboxy-4',5'-dichloro-2',7'-dimethoxyfluoresceine, Yakima Yellow^{™} dyes, Bodipy^{®} 530/550, hexachloro-fluorescein, Alexa Fluor^{®} 555, DY-549, Bodipy^{®} TMR-X, cyanine phosphoramidites (cyanine 3, cyanine 3.5, cyanine 5, cyanine 5.5), ATTO 550, TAMRA (carboxy-tetramethyl-rhodamine), Rhodamine Red^{™}, ATTO 565, Carboxy-X-Rhodamine, Texas Red (Sulforhodamine 101 acid chloride), LightCycler^{®} Red 610, ATTO 594, DY-480-XL, DY-610, ATTO 610, LightCycler^{®} Red 640, Bodipy 630/650, ATTO 633, Alexa Fluor^{®} 647, Bodipy 650/665, ATTO 647N, DY-649, LightCycler^{®} Red 670, ATTO 680, LightCycler^{®} Red 705, DY-682, ATTO 700, ATTO 740, DY-782, IRD 700 and IRD 800, CAL Fluor^{®} Gold 540 nm, CAL Fluor^{®} Gold 522 nm, CAL Fluor^{®} Gold 544 nm , CAL Fluor^{®} Orange 560 nm, CAL Fluor^{®} Orange 538 nm, CAL Fluor^{®} Orange 559 nm, CAL Fluor^{®} Red 590 nm, CAL Fluor^{®} Red 569 nm, CAL Fluor^{®} Red 591 nm, CAL Fluor^{®} Red 610 nm, CAL Fluor^{®} Red 590 nm, CAL Fluor^{®} Red 610 nm, CAL Fluor^{®} Red 635 nm, Quasar^{®} 570 nm, Quasar^{®} 548 nm, Quasar^{®} 566 nm (Cy 3), Quasar^{®} 670 nm, Quasar^{®} 647 nm, Quasar^{®} 670 nm (Cy 5), Quasar^{®} 705 nm, Quasar^{®} 690 nm, Quasar^{®} 705 nm (Cy 5.5), Pulsar^{®} 650 Dyes, SuperRox^{®} Dyes.

In some embodiments, the fluorophore is 6-carboxyfluorescein (6-FAM).

Thus in some embodiments, the nucleic acid is as described above and is labelled in its 5'-end by a fluorophore, such as 6-FAM. The following nucleic acids are thus provided: 6-FAM- AGCTGACCGGCAGCAAAATTG-3', 6-FAM-GAGCTGACCGGCAGCAAAATTG-3'. Any of SEQ ID NOs: 3, SEQ ID NO: 4 or SEQ ID NOs: 6-131 may be labelled with a fluorophore in their 5'-end; in particular embodiments, the fluorophore is 6-FAM.

It will be understood that the fluorophore should be selected such that it is suitable for labelling the 5'-end of a nucleic acid described herein. The 3'-end and the 5'-end of nucleic acid molecules differ in that the 5'-end usually bears a free 5'-phohsphate group, while the 3'-end bears a free 3'-hydroxyl group. Some fluorophores are able to bind only one of the ends (either 3' or 5'), while others are capable of binding both ends. The fluorophores suitable for labelling the nucleic acids described herein are preferably capable of labelling the 5'-end exclusively, or they are capable of labelling the 5'-end and the 3'-end, provided that labelling in the 3'-end does not prevent the labelled nucleic acid from acting as a primer in a reaction for amplifying a target nucleic acid.

### Method for indel detection

The nucleic acids disclosed herein can be used in a method for detecting indels by amplicon analysis.

Thus there is also provided herein a method for detecting an indel in a target nucleic acid, said method comprising the steps of:
a) performing a triprimer amplification reaction to amplify the target nucleic acid, said reaction comprising the steps of:
   i. providing a first elongation primer capable of annealing to a region of a target nucleic acid;
   ii. providing a second elongation primer capable of annealing to another region of said target nucleic acid;
      wherein at least one of the first and the second primers comprises an adaptamer sequence in its 5'-end;
   iii. providing the least one universal primer, said universal primer being labelled by a fluorophore in its 5'-end, and said universal primer being identical to the adaptamer sequence;
      thereby obtaining fluorophore-labelled amplicons;
b) analysing the size of said fluorophore-labelled amplicons in order to determine whether an indel is present.

The inventors have found that the nucleic acids disclosed herein are particularly useful for detecting indels. Indels are, as defined above, insertion or deletion events which can occur for example during gene targeting or gene editing processes, and which result in a net change in the number of nucleotides in a nucleic acid. One of the bottlenecks of many gene editing methods routinely used is the identification of targetants carrying indels. The nucleic acids described herein can be used in a method for identifying indels in a target nucleic acid with a sensitivity of a single nucleotide. The method is based on a triprimer amplification reaction, such as a triprimer PCR. One of the primers is a first elongation primer which is capable to hybridize or anneal to an upstream or downstream region of a target nucleic acid to be amplified. A second elongation primer is capable to hybridize or anneal to another region of the target nucleic acid to be amplified. If the first elongation primer hybridizes to an upstream region, the second elongation primer hybridizes to a downstream region, and vice versa, so that the set of elongation primers can be used in a normal PCR reaction to amplify the target nucleic acid. At least one of the elongation primers comprises an adaptamer sequence, while at most one of the elongation primers hybridizes or anneals to the target nucleic acid sequence substantially over its whole length. At least one other primer is provided which is a universal primer. In some embodiments, the universal primer is a nucleic acid as defined above, and is identical to the adaptamer of at least one of the elongation primers. Enzymes, nucleotides, and other reagents required for amplifying the target nucleic acid are also provided. The amplification reaction is then performed. The end products of the reaction are amplicons which are labelled by the fluorophore. The amplicons are then analysed by suitable methods, such as DNA capillary electrophoresis. The principle of the method is outlined in figure 1. Surprisingly, indels as small as a single nucleotide can be detected when using a universal primer which is a nucleic acid as described herein. Because the method relies on techniques such as PCR which can be performed in a high-throughput manner, it is also suited for high-throughput indel analysis.

Although the above method is illustrated in figure 1 with the first elongation primer hybridizing to the downstream region of the target nucleic acid and the second elongation primer hybridizing to the upstream region of the target nucleic acid, where the second elongation primer comprises an adaptamer sequence, it will be understood that the method can be adapted so the adaptamer is comprised in the elongation primer hybridizing to the downstream region. In this case, the resulting amplicons obtained after amplification are simply labelled in the other end.

It will also be understood that the method can be adapted so that both elongation primers have an adaptamer sequence, which may be identical or different. In some embodiments, the first elongation primer comprises a first adaptamer which has a sequence identical to a first universal primer and the second elongation primer comprises a second adaptamer which has a sequence identical to a second universal primer. The first and second universal primers may differ in sequence. The first and second universal primers may have any of SEQ ID NOs: 3, 4, or SEQ ID NOs: 6-131 as detailed above. The first and second universal primers may be labelled with the same or with different fluorophores.

The method can also be carried out with the first and the second elongation primers comprising the same adaptamer sequence which can be recognised by at least one universal primer. The universal primers recognising the adaptamer sequence may have identical or similar sequences. If the sequences are identical, the universal primers recognising the adaptamer can be labelled with the same fluorophore or with different fluorophores.

The sequence identity between the adaptamer and the universal primer is sufficient to allow hybridization of the universal primer to an amplicon generated with elongation primers where at least one elongation primer comprises the adaptamer. Thus in some embodiments, the sequence identity between the adaptamer and the universal primer is at least 80%, such as at least 81%, such as at least 82%, such as at least 83%, such as at least 84%, such as at least 85%, such as at least 86%, such as at least 87%, such as at least 88%, such as at least 89%, such as at least 90%, such as at least 91%, such as at least 92%, such as at least 93%, such as at least 94%, such as at least 95%, such as at least 96%, such as at least 97%, such as at least 98%, such as at least 99%, such as 100%.

The desirable length of the region of the elongation primers that is capable of hybridizing to the target nucleic acid can be any length suitable for amplifying the target nucleic acid. Thus in some embodiments, the length of the first and/or of the second elongation primers is between 5 and 100 nucleotides, such as between 6 and 90 nucleotides, such as between 7 and 80 nucleotides, such as between 8 and 70 nucleotides, such as between 9 and 60 nucleotides, such as between 10 and 50 nucleotides, such as between 11 and 45 nucleotides, such as between 12 and 40 nucleotides, such as between 13 and 35 nucleotides, such as between 14 and 30 nucleotides, such as between 15 and 25 nucleotides, such as between 16 and 24 nucleotides, such as between 17 and 23 nucleotides, such as between 18 and 22 nucleotides, such as between 19 and 21 nucleotides, such as 20 nucleotides. The optimal length of the hybridizing region of the elongation primers may depend on the sequence of the primers, on the sequence of the target nucleic acid, such as for example their GC content. The skilled person knows how to design suitable elongation primers.

In a preferred embodiment, the at least three primers (two elongation primers and at least one universal primer) are provided simultaneously and the reaction, e.g. the PCR, is performed with all three primers present at the same time as the reaction progresses. In another embodiment, the two elongation primers are provided in a first part of the reaction; this results in a first set of amplicons comprising the adaptamer sequence. The universal primer can thus be provided in a subsequent stage, optionally with additional amounts of at least the elongation primer which hybridizes in the end opposite to the end comprising the adaptamer sequence, and the first set of amplicons resulting from the first step are amplified and labelled in this second step.

The amplification reaction may be any reaction known in the art allowing amplification of a target nucleic acid. Such methods are known in the art, and include PCR, qPCR, RT-PCR, and variations thereof, such as allele-specific PCR, Assembly PCR, Asymmetric PCR, Dial-out PCR, Digital PCR, Helicase-dependent amplification, hot start PCR, intersequence-specific PCR, inverse PCR, ligation-mediated PCR, methylation-specific PCR, multiplex ligation-dependent probe PCR, multiplex PCR, nanoparticle-assisted PCR, nested PCR, overlap-extension PCR, solid phase PCR, suicide PCR, thermal asymmetric interlaced PCR, touchdown PCR and universal fast walking.

The method of the present invention may comprise the step of adding other reagents necessary for performing such amplification reactions. In the case of PCR reaction, such reagents include nucleotides (A, T, G, C), a DNA polymerase, buffer, optionally salts such as magnesium salts, e.g. MgCl₂. Routine optimisation may be required in order to determine the optimal temperature at which the reaction is most efficient and/or specific. PCR reactions are performed in thermal cyclers known in the art.

The invention thus relates to a method of detecting indels, where the universal primer is a nucleic acid as described herein. The universal primer comprises or consists of the sequence 5'-NNNTGACCGGCAGCAAAATTG-3' (SEQ ID NO: 1) or a variant thereof comprising a sequence having at least 85% identity to SEQ ID NO: 5, the 5'-end of said universal primer being labelled with a fluorophore. Thus the universal primer may comprise or consist of any of SEQ ID NO: 3 or SEQ ID NOs: 6 to 68.

In a specific embodiment, the universal primer has the sequence 5'-AGCTGACCGGCAGCAAAATTG-3' (SEQ ID NO: 3) and is labelled at its 5'-end by a fluorophore. In a particular embodiment, the universal primer is SEQ ID NO: 3 labelled with 6-FAM.

In some embodiments, the universal primer is a variant of 5'-NNNTGACCGGCAGCAAAATTG-3' (SEQ ID NO: 1) having at least 85% identity to 5'-TGACCGGCAGCAAAATTG-3' (SEQ ID NO: 5). Thus in one embodiment, the variant comprises a sequence having at least 85% identity, such as at least 86% identity, such as at least 87% identity, such as at least 88% identity, such as at least 89% identity, such as at least 90% identity, such as at least 91% identity, such as at least 92% identity, such as at least 93% identity, such as at least 94% identity, such as at least 95% identity, such as at least 96% identity, such as at least 97% identity, such as at least 98% identity, such as at least 99% identity, such as 100% identity, to SEQ ID NO: 5.

The universal primer may further comprise an additional nucleotide base in the 5'-end. In particular, the additional base may be a guanine base, an adenine base, a thymine base or a cytosine base. In one embodiment, the additional base is a guanine base and the nucleic acid or variant thereof comprises or consists of the sequence 5'-GNNNTGACCGGCAGCAAAATTG-3' (SEQ ID NO: 2). N is any nucleotide base such as a guanine base, an adenine base, a thymine base or a cytosine base. Thus the nucleic acid may comprise or consist of any of SEQ ID NO: 4 or SEQ ID NOs: 69 to 131.

In a specific embodiment, the universal primer comprises or consists of 5'-GAGCTGACCGGCAGCAAAATTG-3' (SEQ ID NO: 4). In a particular embodiment, the universal primer is 5'-GAGCTGACCGGCAGCAAAATTG-3' (SEQ ID NO: 4) and is labelled at its 5'-end by a fluorophore. In a particular embodiment, the universal primer is SEQ ID NO: 4 labelled with 6-FAM.

In some embodiments, the universal primer is a variant of 5'-GNNNTGACCGGCAGCAAAATTG-3' (SEQ ID NO: 2) having at least 85% identity to 5'-TGACCGGCAGCAAAATTG-3' (SEQ ID NO: 5). Thus in one embodiment, the variant comprises a sequence having at least 85% identity, such as at least 86% identity, such as at least 87% identity, such as at least 88% identity, such as at least 89% identity, such as at least 90% identity, such as at least 91% identity, such as at least 92% identity, such as at least 93% identity, such as at least 94% identity, such as at least 95% identity, such as at least 96% identity, such as at least 97% identity, such as at least 98% identity, such as at least 99% identity, such as 100% identity to SEQ ID NO: 5.

The adaptamer comprised within at least one of the elongation primers is identical to the universal primer. Mismatches are possible to the extent that they do not prevent annealing of the universal primer to the adaptamer. Thus in some embodiments, the sequence identity between the adaptamer and the universal primer is at least 80%, such as at least 81%, such as at least 82%, such as at least 83%, such as at least 84%, such as at least 85%, such as at least 86%, such as at least 87%, such as at least 88%, such as at least 89%, such as at least 90%, such as at least 91%, such as at least 92%, such as at least 93%, such as at least 94%, such as at least 95%, such as at least 96%, such as at least 97%, such as at least 98%, such as at least 99%, such as 100%.

The universal primer preferably is not capable of binding or annealing or hybridizing to any sequence of the target nucleic acid or of the DNA material in which the target nucleic acid is comprised or of any DNA material present in the reaction. For example, if the target nucleic acid has been isolated from a cell together with other DNA material, the DNA material and the target nucleic acid do not comprise a sequence identical, complementary to or substantially identical or complementary to the sequence of the universal primer. Without being bound by theory, it is expected that such binding would reduce the efficiency, sensitivity and/or specificity of the present method.

As shown in Example 6, no exact match was found in any of the species of which the genomes are available at NCBI per November 2014 for the nucleic acids having SEQ ID NO: 3 or SEQ ID NOs: 6-68 (see Example 6). Without being bound by theory, it is expected that this is one of the reasons why the present method displays such high specificity.

Routine optimisation may be needed in order to determine the optimal ratio between the first elongation primer, the second elongation primer and the universal primer. By optimal ratio is understood the relative amounts of each primer resulting in optimal reaction specificity and in optimal yield. In some embodiments, the ratio (universal primer):(first elongation primer):(second elongation primer), where the first elongation primer is the primer comprising the adaptamer, is between 1:1:1 and 20:1:20, such as 2:1:2, such as 3:1:3, such as 4:1:4, such as 5:1:5, such as 6:1:6, such as 7:1:7, such as 8:1:8, such as 9:1:9, such as 10:1:10, such as 11:1:11, such as 12:1:12, such as 13:1:13, such as 14:1:14, such as 15:1:15, such as 16:1:16, such as 17:1:17, such as 18:1:18, such as 19:1:19, such as 2:1:3, such as 3:1:4, such as 4:1:5, such as 5:1:6, such as 6:1:7, such as 7:1:8, such as 8:1:9, such as 9:1:10, such as 10:1:11, such as 11:1:12, such as 12:1:13, such as 13:1:14, such as 14:1:15, such as 15:1:16, such as 16:1:17, such as 17:1:18, such as 18:1:19, such as 19:1:20, such as 3:1:2, such as 4:1:3, such as 5:1:4, such as 6:1:5, such as 7:1:6, such as 8:1:7, such as 9:1:8, such as 10:1:9, such as 11:1:10, such as 12:1:11, such as 13:1:12, such as 14:1:13, such as 15:1:14, such as 16:1:15, such as 17:1:16, such as 18:1:17, such as 19:1:18, such as 20:1:19. As the skilled person knows, the optimal primer ratio is dependent on many parameters, including the exact sequence of the elongation primers, which influences the hybridization temperature and the possible formation of secondary structures which might interfere with the reaction efficiency. Another parameter may be the nature of the target nucleic acid: some target regions are more easily accessible to primers than others depending of their environment or on the secondary structures they may adopt. Without being bound by theory, the inventors have found that it is often advantageous that the primer which comprises the adaptamer sequence be present in a smaller amount than the other elongation primer and the universal primer, which themselves are preferably present in equimolar amounts. Such ratios have been observed to increase specificity of the reaction.

Other parameters such as the number of cycles or the temperatures of the different steps of the amplification reaction may also need routine optimisation. The skilled person knows how to optimise such parameters.

After the amplification reaction has been performed, the size of the fluorophore-labelled amplicons thus obtained is analysed.

Methods for analysing the size of the labelled amplicons are available to the skilled person. For example, the fluorophore-labelled amplicons are detected by DNA capillary electrophoresis (CE), melting curve analysis, polyacrylamide gel electrophoresis or other size exclusion methods where laser induced fluorophore detection can be applied. Suitable equipment for analysing amplicon size is known to the skilled person and includes e.g. genetic analysers.

The method described herein can detect insertions or deletions that are very small, i.e. down to a single nucleotide insertion or deletion. Thus in one embodiment, the method allows detection of an indel of at the most 10 nucleotides, such as 9 nucleotides, such as 8 nucleotides, such as 7 nucleotides, such as 6 nucleotides, such as 5 nucleotides, such as 4 nucleotides, such as 3 nucleotides, such as 2 nucleotides, such as 1 nucleotide at the most.

The method of the present invention is thus particularly well suited for high-throughput analysis of targetants following gene editing or gene targeting. Methods of gene editing or gene targeting are known in the art and include, but are not limited to: zinc-finger endonuclease (ZFN) editing; TALEN-mediated editing; CRISPR-Cas-based methods; targeted mutagenesis using primers comprising the desired mutations; random mutagenesis; integrative plasmids. Such gene editing or targeting methods sometimes result in off-target effects and/or creation of indels and/or other undesirable effects, as explained above.

### Target nucleic acid

The target nucleic acids to be analysed using the present method may be of several kinds. In some embodiments, the target nucleic acid is comprised or has been comprised within a cell, for example in a genome of a cell, or on a plasmid. In some embodiments, the target nucleic acid is a viral nucleic acid.

In particular embodiments, the cell is a eukaryotic cell. The eukaryotic cell may be selected from the group consisting of a human cell, a Chinese hamster cell, a murine cell, a rat cell, an insect cell such as an Sf9 cell, a canine cell, a plant cell, an old world monkey cell, a new world monkey cell, a pig cell, a horse cell, a bovine cell, a goat cell, a lamb cell, a fish cell, an avian cell, a feline cell and a yeast cell such as *Saccharomyces cerevisiae, Schizosaccharomyces pombe,* or *Pichia pastoris.*

In other embodiments, the eukaryotic cell is a plant cell. The plant cell may be from a genus such as the family Brassicaceae such as *Arabidopsis thaliana,* the *Solanaceae,* or nightshade genus such as tomato, *Solanum tuberosumor* potato, cereal grain such as rice or wheat, corn or maize or other.

In other embodiments, the cell is a prokaryotic cell. The prokaryotic cell may be a bacterial cell. The cell may originate from a bacteria selected from the group of *Escherichia sp.* such as *E. coli*, *Lactobacillus sp., Streptomyces sp. Campylobacter sp., Salmonella sp., Listeria sp., Staphylococcus sp. Bacillus sp.,* and *Clostridium sp.*

In some embodiments, isolation of the DNA material of the cell comprising the target nucleic acid is required. Methods for isolating DNA from cells are known in the art. In other embodiments, the amplification reaction is performed directly on cell samples without prior DNA extraction. In such embodiments, cells may be lysed prior to amplification, as is known to the skilled person. Lysis can be performed by incubating the cells at high temperatures, or by using lysing agents known in the art. Whether DNA extraction is necessary will depend on factors such as e.g. the nature of the cell and the nature of the target nucleic acid.

In specific embodiments, the cell comprising the target nucleic acid to be analysed with the present method is comprised or has been comprised within a pool of cells, for example a pool of targetants. The targetants within the pool may be genetically identical (i.e. propagated from a single cell or clone) or different.

In some embodiments, the present method is suitable for high-throughput screening of targetants.

Thus the present method is useful for screening targetants obtained after gene or genome editing. The targetants may be the result of strategies involved methods of genome editing known in the art. In some embodiments, the targetants are obtained after cloning using transfection with DNA fragments having homology to a target region. In other embodiments, the targetants are obtained after random mutagenesis. In yet other embodiments, integrative plasmids are used. In yet other embodiments, the genome editing is performed using ZFNs, TALENS or CRISPR systems. It is to be understood that the present method is not limited to targetants obtained by particular gene editing strategies.

The present method is also useful for determining gene targeting efficiency.

The present invention thus relates to a nucleic acid as defined above for use in a method of detecting an indel in a target nucleic acid.

In some embodiments, the indel is an insertion and/or a deletion resulting in a net change of the total number of nucleotides within a nucleic acid, where the net change is equal to m, where m is an integer ≥ 1. Thus in specific embodiments, the desired size of the target nucleic acid (unmodified) is S and the indel is an insertion, so that the size of the nucleic acid amplified with the present method is S + m, wherein m is at least 1, such as at least 2, such as at least 3, such as at least 4, such as at least 5, such as at least 6, such as at least 7, such as at least 8, such as at least 9, such as at least 10. In other specific embodiments, the indel is a deletion and the size of the nucleic acid amplified with the present method is S - m, wherein m is at least 1, such as at least 2, such as at least 3, such as at least 4, such as at least 5, such as at least 6, such as at least 7, such as at least 8, such as at least 9, such as at least 10. Accordingly, in some embodiments, the nucleic acids of the present invention can be used in a method of detecting an indel in a target nucleic acid, where the indel is an insertion or a deletion of 1 nucleotide or more, such as at least 2 nucleotides, such as at least 3 nucleotides, such as at least 4 nucleotides, such as at least 5 nucleotides, such as at least 6 nucleotides, such as at least 7 nucleotides, such as at least 8 nucleotides, such as at least 9 nucleotides, such as at least 10 nucleotides, or more. Detecting indels in the resulting products with the method described herein has numerous potential applications, such as, but not limited to, diagnosing a disease or a disorder. As an example, the nucleic acids disclosed herein can be used for detecting microsatellite expansion, contraction, trinucleotide repeat disorders. Trinucleotide repeat disorders are typically due to trinucleotide repeat expansion events, and comprise polytglutamine diseases, wherein the glutamine-encoding codon CAG is repeated, and non-polyglutamine diseases, which involve repeats of other trinucleotides.

Examples of diseases or disorders resulting from such mechanisms are Dentatorubropallidoluysian atrophy, Huntington's Disease, spinal and bulbar muscular atrophy, spinocerebellar ataxia type 1, 2, 3, 6, 7, 8, 12 or 17, fragile X syndrome, Fragile X-associated tremor/ataxia syndrome, fragile XE mental retardation, Friedreich's ataxia and myotonic dystrophy.

### Methods for synthesis

Outside the scope of the invention the disclosure relates to a method for synthesizing a nucleic acid of the

### invention.

Methods for synthesizing nucleic acids as disclosed herein are known to the skilled person. Such methods include, but are not limited to, chemical oligonucleotide synthesis methods such as solid-phase synthesis. The synthetic oligonucleotides can then be released from the solid phase to solution and collected.

Methods for fluorescently labelling the synthetic oligonucleotides are also known to the skilled person. In the present context, fluorescent labelling is the process of covalently attaching a fluorophore to a nucleic acid. This is typically accomplished using a reactive derivative of the fluorophore that selectively binds to a functional group contained in the target molecule. Common reactive groups include, but are not limited to: isothiocyanate derivatives such as FITC and TRITC (derivatives of fluorescein and rhodamine), succinimidyl esters such as NHS-fluorescein, maleimide activated fluorophores such as fluorescein-5-maleimide, or phosphoramidite reagents containing protected fluorescein and other fluorophores, e.g. 6-FAM phosphoramidite 2. Phosphoramidte agents are can be reacted with hydroxy groups to allow the preparation of fluorophore-labelled oligonucleotides.

### Kit

Outside the scope of the claimed invention is provided herein a kit comprising a nucleic acid as defined herein and instructions for use. The kit may further comprise reagents required for performing an amplification reaction and/or detecting indels and/or for performing the method of the invention.

### Examples

### Example 1. Materials and methods

### Precise gene targeting induced indel detection by amplicon analysis (IDAA)

All primers used were obtained from TAGC Copenhagen A/S, Denmark. Amplicons were fluorophore labeled by tri-primer amplification using a universal 6-FAM 5'-labelled primer FamF and primers flanking the gene editing target site of which the sense primer carried a FamF target sequence extension. For each of the following genes, the universal primer had the sequence AGCTGACCGGCAGCAAAATTG (SEQ ID NO: 3): hCOSMC, mCosmc, cCosms, *hGALNT6*, *cSt6galnac2*, hKRAS,

Optimal tri-primer generated amplicon yields were observed using a PCR primer ratio of 10:1:10 (FamF:XF:XR), X being either hCOSMC, *hGALNT6, hKRAS,* mCosmc, *cSt6galnac2* or cCosmc. PCR was performed in 25 µl, using AmpliTaq Gold (ABI/Life Technologies, USA) or TEMPase Hot Start DNA Polymerase (Amplicon, Denmark), 0,5 µM:0.05 µM:0.5 µM (FamF:F:R) primers and a touchdown thermocycling profile using an initial 72°C annealing temperature ramping down by 1 degree/cycle to 58°C, followed by an additional 25 cycles using 58°C annealing temperature. Denaturing and elongation were performed at 95°C for 45 sec and 72°C for 30 sec respectively. 1 µl of the PCR reaction or dilutions thereof was mixed with 0.5 µl LIZ600 or LIZ500 size standard (ABI/Life Technologies, USA) and applied to fragment analysis on ABI3010 sequenator (ABI/Life Technologies, USA) using conditions recommended by the manufacturer. Raw data obtained was analysed using Peak Scanner Software V1.0 (ABI/Life Technologies, USA).

### Gene targeting plasmids and dual ZFN plasmid construction

CompoZr ^{®} ZFN plasmids for human *C1GALT1C1*/*COSMC*, mouse *C1galt1c1*/*Cosmc* and human *GALNT6* were obtained from Sigma (Sigma-Aldrich, St. Louis, MO, USA). GeneArt^{®} TALEN *St6galnac2* CHO plasmids were obtained LifeTechnologies (Thermo Fisher Scientific Inc, Waltham, MA, USA). Cas9 plasmid was codon optimized for CHO expression. Four CHO Cosmc gRNA targets were selected using a tool developed for Cas9/gRNA target prediction (staff.biosustain.dtu.dk/laeb/crispy/). Dual *GALNT6* expression vector was constructed as follows: GFP and the two *GALNT6* ZFN1/2 sequences were fused via 2A peptide as outlined in Fig. 4a. In brief, a sequence encoding GFP, Flag and nuclear localization signal (NLS) was inserted in frame into the EcoRl/Kpnl site of the CompoZr ^{®} *GALNT6* ZFN-2 plasmid leaving GFP fused via 2A peptide, Flag and NLS to the *GALNT6* ZFN-2 ORF as described. A full sequence of codon optimized *GALNT6* ZFN-1 (Genewiz, South Plainfield, NJ, USA) fused to sequences encoding 2A peptide, 2xmyc tag and a nuclear localization signal (NLS) was directionally inserted into the Kpnl site, generating GFP-2A-*GALNT6*-ZFN-1-2A-ZFN-2 (Dual-GALNT6-ZFN). All plasmids were Sanger sequenced. Expression and cleavage of ZFNs were verified by SDS-PAGE Western analysis using immune reagents to the GFP, ZFN1 (Flag-tag), and ZFN2 (myc-tag) as illustrated in Fig.4b.

### Cell culture, transfections and FACS sorting

HeLa, DE4, HEK293AC2, mouse MC57 cells were cultured in DMEM with 10% FBS and 1% L-glutamine, and K562 cells were cultured in Iscove's modified Dulbecco's medium, 10% FBS and 1% L-glutamine. CHO cells were cultured in ex-Cell-CD media (Sigma Aldrich, USA) with 2% L-glutamine. Cells were nucleofected using solution kits T and V (K562) (Lonza, USA) and a Amaxa ^{®} Cell Line Nucleofector^{®} device as previously described using protocols provided by Lonza. In brief, 1 × 10⁶ cells were transfected with 2 µg of ZFN or TALEN plasmid pairs or 2 µg of Dual-GALNT6-ZFN. For CRISPR/Cas9 CHO *Cosmc* targeting, 2 µg Cas9 and gRNA plasmids were nucleofected, and for pCMV-Cas9-GFP expressing *KRAS* gRNA 2 µg plasmid was used. Cells were exposed to a cold shock 30°C for 2 days post-transfection, and incubated one day at 37°C after which DNA of the cell pool was prepared using Nucleospin kit as recommended by the supplier (Machery-Nagel, USA).

For consecutive targeting of the *KRAS* locus, K562 cells were subjected to FACS 3 days after nucleofection for isolation of the 2% most highly GFP fluorescent cells that were then cultured for about 1 week. Thereafter, an aliquot of the cell pool was analysed by IDAA (1^{st} hit), whereas the rest of the cells were subjected to another two rounds of nucleofection and FACS to produce 2^{nd} and 3^{rd} hit pools, respectively, Furthermore, after the 3^{rd} hit, cells were also single-cell plated in 96-well plates and expanded to clonal cell lines.

### Direct Sanger sequencing

Expanded single cell clones were lysed in the wells using QuickExtract DNA extraction solution (Sigma-Aldrich, USA), 1 µl lysate was used for target region amplification. Amplified products were band purified using Qia-mini elute purification (Qiagen Inc, USA) and used for Topo-ligation into pCR4-Topo vector (Invitrogen/Life Technologies, USA), transformed into MegaXcells (Invitrogen/Life Technologies, USA) and LB-Streptomycin/Carabenicillin plated. A custom based direct sequencing protocol was developed by which large sized single cell colonies were boiled in 10 µl TE for 10 min and 5 µl hereof added to BigDye.3.1 reaction (ABI/Life Technologies, USA) and sequenced using 45x sequencing cycles (BGI Europe, Denmark).

### T7El-nuclease assay

Endonucleolytic heteroduplex DNA cleavage analysis was performed using T7-nuclease-I (New England Biolabs, USA) as recommended by the supplier. In brief, heteroduplex and/or perfect match amplicons were incubated with 1 µl T7nuclease in a 20 µl volume at 37°C for 1 h, followed by 3% agarose gel GelStar (Lonza, USA) analyses.

### SDS-PAGE Western blotting and immunocytochemistry

K562 cells were nucleofected as described above. After 1, 2 or 5 days, aliquots of the cells were harvested and lysed in SDS-PAGE sample buffer. The cell lysates were normalized for protein content and equal amounts of protein were subjected to immunoblotting. Blots were incubated with primary antibodies to the proteins indicated (beta-Actin: Abcam, cat. number: ab8226; c-*Myc*: Santa Cruz Biotechnology, cat. number: sc-40; Flag: Sigma-Aldrich, cat. number: F3165) followed by HRP-conjugated secondary antibodies (Dako, Denmark), both for 1 hr at room temperature and finally developed with ECL (Pierce/Thermo Scientific, USA). For immunocytochemistry (ICC), CHO cells were fixed and stained on Teflon coated slides. In brief, cells were dried on slides and incubated overnight, 4°C, with the monoclonal antibody 5F4, followed by secondary anti-mouse-Ig-FITC incubation (Dako, Denmark), visualization and imaging by fluorescence microscopy.

### Example 2. IDAA for estimating cutting efficiencies for CRISPR/Cas9-gRNA designs

We have developed a single-step tri-primer PCR setup with a universal 6-FAM 5'-labelled primer (FamF), which is designed to be specific for an extension placed on one of the target specific primers, and thus enabling one-step fluorophore labelling of amplicons derived from any given target using a universal single set-up condition (Fig. 1a). The tri-primer amplification assay was standardized and optimized for general use in detecting a broad range of targets. Optimal amplicon yields were obtained using 10:1:10 molar ratios of 5'-labelled primer:elongation primer:reverse primer (Fig. 1b). The fluorophore labelled amplicons can easily be detected with great sensitivity and with accurate size determination using standard DNA fragment analysis by capillary electrophoresis methodology (Fig. 1c).

To demonstrate the applicability of the IDAA strategy in nuclease-based genome editing, we first demonstrated its use for evaluating cutting efficiencies of CRISPR/Cas9 targeting using four different gRNA designs (Fig. 2a). We targeted the *Cosmc* gene (X-linked) in Chinese hamster ovary (CHO) cells, because CHO only has one *Cosmc* allele and we have a very reliable phenotypic screen for knockout of *Cosmc* function (Steentoft et al., 2011). CHO-GS ells were maintained as suspension cultures in EX-CELL CHO CD Fusion serum and animal component free media, supplemented with 4 mM L-glutamine. The cells were seeded at 0.5 × 10⁶ cells/mL in T25 flask (NUNC, Denmark) one day prior to transfection. 2×10⁶ cells and 2 µg endotoxin free plasmid encoding codon optimized Cas9 and either one of four gRNA designs specific for CHO *Cosmc* are transfected by electroporation using Amaxa kit V and program U24 with Amaxa Nucleofector 2B (Lonza, Switzerland). ). Subsequently, cells were diluted in 3 mL growth media, seeded in a well of a 6-well plate, incubated at 30°C for a 24 h "cold shock" period followed by 37°C incubation for an additional 24 h. These cells are designated the cell pool. The 10-15% highest GFP fluorescing cells in the cell pool were enriched by FACS. FACS sorting was performed again one week after enrichment to obtain single clones in round bottom 96 well plates.

IDAA analysis of the four cell pools obtained after CRISPR/Cas) gRNA 2 days post transfection shows that the total cutting efficiencies for gRNA1 and gRNA2 are 23% and 46%, respectively, see Fig 2a, with the +1bp found to be the major indel, while gRNA3 and gRNA4 were inactive. Representative IDAA analysis of single cell clones can be seen in Fig 2e, showing the 1 bp resolution power of IDAA.

We found consistent targeting efficiencies of total cell pools when using either IDAA or phenotypic screening 3d after transfection with the constructs (Fig 2b). The IDAA analysis of the total cell pools and Sanger sequenced single cell clones furthermore revealed that indels ranged from +1 to -13 with an apparent frequency of <1% to a few percent for most of them (Fig. 2a and 2c). Notably the identity of the predominant +1 (approximate frequency 20% and 32% for gRNA2 and gRNA3 respectively) insertion was validated by Sanger and found to be a T/A base-pair insertion (Fig. 2d).

Taken together the results demonstrate the usefulness of IDAA in determining cutting efficiencies of gene targeting tools such as CRISPR/Cas9, ZFN, TALEN or other in cell pools and single cell clones, down to +/-1bp differences. The example in particular demonstrates the usefulness of IDAA in determining the efficacies of gRNA designs with unknown CRISPR/Cas9 cutting efficacy and as such IDAA is ideally suited for validating the efficiency of a multitude of gRNA designs.

### Example 3. Comparison of IDAA against Sanger sequencing and enzyme mismatch cleavage assay

We next demonstrate that IDAA is amenable for high throughput screening of targeted individual cell clones with a discrimination power down to a single base indel. We used the same CRISPR/Cas9 *Cosmc* gRNA2 targeted pool for single cell cloning shown in Figure 2a, and analysed approximately 200 independent single cell clones by Sanger and IDAA. Notably, the -5bp to +5bp indel distribution obtained by MiSeq next generation deep sequencing (Fig. 10) and Sanger was similar to the IDAA distribution on the cell pool (Fig. 2a and 2c). A variety of distinct clones with indels ranging from - 74bp to +31 bp were readily identified by IDAA, as exemplified in Fig. 2e, which also illustrates the 1 bp resolution power of IDAA for indel genotyping of clones and the ease by which clones harbouring reading frame-disrupting indels can be identified. The indels were confirmed by Sanger sequencing as shown for the predominant +1 insertion identified (Fig. 2d).

To corroborate the usability of IDAA for multi-allele gene targeting, bi-allelic CHO *St6galnac2* and tri-allelic human K562 *KRAS* were targeted with TALEN and CRISPR/Cas9 nucleases respectively. The IDAA results for TALEN CHO *St6galnac2* two days after transfection clearly detected indels in the cell pool and successful bi-allelic targeting was obtained in a substantial fraction (48%) of FACS single cell clones analysed (Fig. 5a and 5b). For CRISPR/Cas9 human K562 *KRAS* targeting, FACS for the 2% most highly fluorescent cells generated a cell pool (1^{st} hit) in which IDAA revealed indels in the large majority of alleles and only a minor wt peak (Fig. 6a). When this pool was subjected to further two consecutive rounds of targeting and FACS, IDAA revealed near-complete modification of the *KRAS* locus, since the wt peak was hardly detectable. By contrast, these large modification rates were greatly underestimated by EMC assay (Fig. 6b). Sanger sequencing and IDAA analysis of 96 single cell clones isolated from the 3^{rd} hit pool confirmed the indel profile obtained by IDAA on the 3^{rd} hit cell pool and successful tri-allelic targeting in 99% of the clones was detected by IDAA (only 1 wt allele was detected) (Fig. 6c and 6d). The IDAA clone analysis in Fig. 5b and 6d shows that clones harboring reading frame-disrupting indels in all alleles present can be identified. By contrast, no such information can be derived from EMC assay which even fails to detect complete allele modification in clones, as illustrated in Fig. 6b.

We further showed that the IDAA strategy is ideal for ZFNs, which generally have lower cutting efficiencies. We first tested a ZFN with medium cutting efficiency (18% evaluated by an EMC assay, Sigma-Aldrich) targeting the *Cosmc* gene in a murine cell line (MC57) (Fig. 3). We used a GFP-tagged ZFN approach that enables FACS sorting for enrichment of targeting events as recently described (Duda et al., 2014). A total of 81 single cell clones derived from 192 FACS seeded wells were tested by IDAA, and we found complete correlation between the IDAA results and Sanger sequencing. We then tested a ZFN with low cutting efficiency (2.8% as determined by an EMC assay, Sigma-Aldrich) targeting the human *GALNT6* gene in human HepG2 cells (Fig. 4). For this we used a novel dual expression plasmid encoding both ZFNs fused to GFP (Dual-GALNT6-ZFN). We used IDAA to analyze the cell pool at day two post-transfection, and we could demonstrate that the predominant targeting events were +/- 1bp indels detectable with around only 1% frequencies. These predominant low targeting events could also be detected by MiSeq deep sequencing (Fig. 10b), but such patterns of targeting events with predominantly small indels are not expected to be detectable by the traditional EMC assays. To test this hypothesis, we performed a head-to-head comparison of IDAA with the most commonly used indel detection assay. The EMC assay has been shown to display a preference for heteroduplex DNA formed by larger deletions rather than single 1 base indels, and it does not provide information as to the nature of the indels and types of alleles present. We first demonstrated that the EMC assay using T7 endonuclease I easily detected a large deletion, in this instance induced by a ZFN (Fig. 7a). We next tested the EMC assay on a COSMC targeted cell clone with only one allele and possessing a single base indel. In this case amplicons derived from the HeLa targeted COSMC allele (Steentoft et al., 2013) and wild type HeLa cells were mixed and analyzed, and we confirm that whereas larger DNA deletions are easily detected by EMC, a single base indel is not (Fig. 7b).

These data show that IDAA can successfully be used to detect indels as small as 1 bp, even when such events occur at low frequencies, while they are not detected by the EMC assay.

### Example 4. IDAA for detection of candidate off-target indels

We demonstrate that IDAA is ideally suited for fast and simple screening for indels in candidate off-target genes identified by target sequence similarity, which is a major concern for all precise gene editing strategies. We first screened the CHO genome for the most likely off-target sites for gRNA2 targeting *Cosmc*, and identified a total of 189 potential off-targets with two to four bp mismatches (Fig. 8a). Detailed mismatch distribution for the top 21 off targets are shown in fig. 11. We tested the most likely off-target (2 mismatches, no candidate genes for gRNA2 with only one mismatch were found) in 10 independent *Cosmc* Cas9/gRNA2 targeted single cell clones by IDAA and we could demonstrate complete absence of off-target events in all clones (Fig. 8b). We next screened another 20 most likely off-targets (3-4 mismatches) in all 10 clones, and again confirmed complete absence of off-target events in all 10 clones (Fig. 9). It should be stressed that our analysis was conducted on independent cell clones and not on cell pools where rare off-target effects could be expected.

This example shows that IDAA can be used to demonstrate the absence of off-target events in clones to be tested.

### Example 5. Detection of stable, heritable, precise gene edited indels

The present IDAA method is ideally suited for the detection of stable, heritable precise gene edited indels in target cells to be used for therapeutic purposes.

This example demonstrates IDAA's use in investigation and identification of ZFN-mediated indels at the *CXCR4* locus in primary T cells. Fresh CD4+ T cells from live human donors are obtained from Blodbanken, Ålborg Sygehus Nord, Denmark. 2,5 × 10⁶ CD4+ cells are seeded at a density of 0,8 × 10⁶ in RPMI containing 10% fetal calf serum, 1% penicillin/streptomycin, and 100U/ml interleukin-2. 1 × 10⁶ cells are nucleofected with 2 µg of each of the *CXCR4* ZFN (CompoZr^{®} Knockout ZFN Kit, Sigma-Aldrich, USA) using Amaxa nucleofector (Lonza, USA) using protocols and reagents as described by the manufacturer. Cells are treated essentially as explained in example 1. Two days post transfection the *CXCR4* treated cell pool is examined by IDAA for quantification of targeting efficiency as decribed in example 2. Tri-primer *CXCR4* target specific primers; *CXCR4* extension primer (5'-GAGCTGACCGGCAGCAAAATTGCAACCTCTACAGCAGTGTCCTCATC-3' (SEQ ID NO: 133) adaptamer sequence underlined)/*CXCR4REV* (5'-GGAGTGTGACAGCTTGGAGATG-3' and universal primer (SEQ ID NO: 3) were used to fluorophore label *CXCR4* amplicons for IDAA as described in example 1. Mutation frequencies obtained by IDAA match the described frequencies obtained by *CXCR4* ZFN manufacturer (Sigma-Aldrich). Cells abrogated for *CXCR4* expression due to *CXCR4* targeting will be resistant to natural *CXCR4*-tropic HIV strains as described in (CA Didigu et al., 2014, Blood, 123, p61-69) and as such, the percent of *CXCR4* gene disrupted population increases after *CXCR4*-tropic HIV infection due abrogated *CXCR4* coreceptor expression blocking the ability of X4-tropic virus to infect cells and conferring a survival advantage to the cell population modified by the *CXCR4*-specific ZFNs. Such HIV-resistant CD+ T cells are to be considered for therapeutic treatment of HIV infected individuals.

This example demonstrates that the present method and nucleic acids can be used for therapeutic purposes.

In conclusion, the IDAA strategy presented here enables sensitive, precise and reliable identification of indels in a high throughput mode providing detailed information of cutting efficiency, size and nature of allelic variants generated by any of the precise gene editing technologies. The IDAA strategy is user friendly and easily implemented in any standard laboratory and can greatly advance implementation and use of precise gene targeting.

### Example 6. Blast analysis

SEQ ID NO: 4 and variants thereof (SEQ ID NOs: 134-187, SEQ ID NOs: 71, 75, 79, 81-83, 98, 114, 130) were analysed as follows. All positions except for the first G-nucleotide were substituted for any of the 4 bases (A, T, G and C) and all individual permutations were BLAST analysed against the total species NCBI database as of November 2014. No exact matches were found in any of the species.

Mismatch hits and unique number of taxa hits (taxa being any group or rank in a biological classification into which related organisms are classified) were analysed in human and mouse; mismatches were allowed at any position and SEQ ID NO: 4 was used as query. The results are shown in table 1:

**Table 1.**

| Mismatch | number | % similarity | unique taxa hits | human/mouse |
|---|---|---|---|---|
| 1 | 62 | 95 | 19 | 0 |
| 2 | 1240 | 90 | 206 | yes |
| 3 | 3448 | 86 | 522 | yes |
| 4 | 2437 | 82 | 496 | yes |

As can be seen for queries with only one mismatch, no identical sequences were found.

When allowing 1 to 4 mismatches, 917 unique taxa hits were retrieved (data not shown).

SEQ ID NO: 3 and variants thereof (SEQ ID NOs: 188-241 and SEQ ID NOs: 8, 12, 16, 18-20, 35, 51, 67) were analysed as follows. All positions were substituted for any of the 4 bases (A, T, G and C) and all individual permutations were BLAST analysed against the total species NCBI database as of November 2014. No exact or 1 bp matches were found in any of the species.

Mismatch hits and unique number of taxa hits (taxa being any group or rank in a biological classification into which related organisms are classified) were analysed in human and mouse; mismatches were allowed at any position and SEQ ID NO: 3 was used as query. The results are shown in table 2:

**Table 2.**

| Mismatch | number | % similarity | unique taxa hits | human/mouse |
|---|---|---|---|---|
| 2 | 48 | 90 | 26 | 0 |
| 3 | 2210 | 86 | 284 | yes |
| 4 | 4502 | 81 | 403 | yes |

As can be seen for queries with one or two mismatches, no identical sequences were found.

When allowing 2 to 4 mismatches, 472 unique taxa hits were retrieved (data not shown).

**Table 3. Sequences.**

| **SEQ ID** | **Description** | **Sequence** |
|---|---|---|
| SEQ ID NO: 1 | Universal primer | nnntgaccgg cagcaaaatt g |
| SEQ ID NO: 2 | Extended universal primer | gnnntgaccg gcagcaaaat tg |
| SEQ ID NO: 3 | FAMFOR primer | agctgaccgg cagcaaaatt g |
| SEQ ID NO: 4 | Extended FAMFOR primer | gagctgaccg gcagcaaaat tg |
| SEQ ID NO: 5 | Core sequence | tgaccggcag caaaattg |
| SEQ ID NO: 6 | Variant 3 | aaatgaccgg cagcaaaatt g |
| SEQ ID NO: 7 | Variant 4 | aagtgaccgg cagcaaaatt g |
| SEQ ID NO: 8 | Variant 5 | aactgaccgg cagcaaaatt g |
| SEQ ID NO: 9 | Variant 6 | aattgaccgg cagcaaaatt g |
| SEQ ID NO: 10 | Variant 7 | atatgaccgg cagcaaaatt g |
| SEQ ID NO: 11 | Variant 8 | atgtgaccgg cagcaaaatt g |
| SEQ ID NO: 12 | Variant 9 | atctgaccgg cagcaaaatt g |
| SEQ ID NO: 13 | Variant 10 | atttgaccgg cagcaaaatt g |
| SEQ ID NO: 14 | Variant 11 | acatgaccgg cagcaaaatt g |
| SEQ ID NO: 15 | Variant 12 | acgtgaccgg cagcaaaatt g |
| SEQ ID NO: 16 | Variant 13 | acctgaccgg cagcaaaatt g |
| SEQ ID NO: 17 | Variant 14 | acttgaccgg cagcaaaatt g |
| SEQ ID NO: 18 | Variant 15 | agatgaccgg cagcaaaatt g |
| SEQ ID NO: 19 | Variant 16 | aggtgaccgg cagcaaaatt g |
| SEQ ID NO: 20 | Variant 17 | agttgaccgg cagcaaaatt g |
| SEQ ID NO: 21 | Variant 18 | taatgaccgg cagcaaaatt g |
| SEQ ID NO: 22 | Variant 19 | tagtgaccgg cagcaaaatt g |
| SEQ ID NO: 23 | Variant 20 | tactgaccgg cagcaaaatt g |
| SEQ ID NO: 24 | Variant 21 | tattgaccgg cagcaaaatt g |
| SEQ ID NO: 25 | Variant 22 | ttatgaccgg cagcaaaatt g |
| SEQ ID NO: 26 | Variant 23 | ttgtgaccgg cagcaaaatt g |
| SEQ ID NO: 27 | Variant 24 | ttctgaccgg cagcaaaatt g |
| SEQ ID NO: 28 | Variant 25 | ttttgaccgg cagcaaaatt g |
| SEQ ID NO: 29 | Variant 26 | tcatgaccgg cagcaaaatt g |
| SEQ ID NO: 30 | Variant 27 | tcgtgaccgg cagcaaaatt g |
| SEQ ID NO: 31 | Variant 28 | tcctgaccgg cagcaaaatt g |
| SEQ ID NO: 32 | Variant 29 | tcttgaccgg cagcaaaatt g |
| SEQ ID NO: 33 | Variant 30 | tgatgaccgg cagcaaaatt g |
| SEQ ID NO: 34 | Variant 31 | tggtgaccgg cagcaaaatt g |
| SEQ ID NO: 35 | Variant 32 | tgctgaccgg cagcaaaatt g |
| SEQ ID NO: 36 | Variant 33 | tgttgaccgg cagcaaaatt g |
| SEQ ID NO: 37 | Variant 34 | caatgaccgg cagcaaaatt g |
| SEQ ID NO: 38 | Variant 35 | cagtgaccgg cagcaaaatt g |
| SEQ ID NO: 39 | Variant 36 | cactgaccgg cagcaaaatt g |
| SEQ ID NO: 40 | Variant 37 | cattgaccgg cagcaaaatt g |
| SEQ ID NO: 41 | Variant 38 | ctatgaccgg cagcaaaatt g |
| SEQ ID NO: 42 | Variant 39 | ctgtgaccgg cagcaaaatt g |
| SEQ ID NO: 43 | Variant 40 | ctctgaccgg cagcaaaatt g |
| SEQ ID NO: 44 | Variant 41 | ctttgaccgg cagcaaaatt g |
| SEQ ID NO: 45 | Variant 42 | ccatgaccgg cagcaaaatt g |
| SEQ ID NO: 46 | Variant 43 | ccgtgaccgg cagcaaaatt g |
| SEQ ID NO: 47 | Variant 44 | ccctgaccgg cagcaaaatt g |
| SEQ ID NO: 48 | Variant 45 | ccttgaccgg cagcaaaatt g |
| SEQ ID NO: 49 | Variant 46 | cgatgaccgg cagcaaaatt g |
| SEQ ID NO: 50 | Variant 47 | cggtgaccgg cagcaaaatt g |
| SEQ ID NO: 51 | Variant 48 | cgctgaccgg cagcaaaatt g |
| SEQ ID NO: 52 | Variant 49 | cgttgaccgg cagcaaaatt g |
| SEQ ID NO: 53 | Variant 50 | gaatgaccgg cagcaaaatt g |
| SEQ ID NO: 54 | Variant 51 | gagtgaccgg cagcaaaatt g |
| SEQ ID NO: 55 | Variant 52 | gactgaccgg cagcaaaatt g |
| SEQ ID NO: 56 | Variant 53 | gattgaccgg cagcaaaatt g |
| SEQ ID NO: 57 | Variant 54 | gtatgaccgg cagcaaaatt g |
| SEQ ID NO: 58 | Variant 55 | gtgtgaccgg cagcaaaatt g |
| SEQ ID NO: 59 | Variant 56 | gtctgaccgg cagcaaaatt g |
| SEQ ID NO: 60 | Variant 57 | gtttgaccgg cagcaaaatt g |
| SEQ ID NO: 61 | Variant 58 | gcatgaccgg cagcaaaatt g |
| SEQ ID NO: 62 | Variant 59 | gcgtgaccgg cagcaaaatt g |
| SEQ ID NO: 63 | Variant 60 | gcctgaccgg cagcaaaatt g |
| SEQ ID NO: 64 | Variant 61 | gcttgaccgg cagcaaaatt g |
| SEQ ID NO: 65 | Variant 62 | ggatgaccgg cagcaaaatt g |
| SEQ ID NO: 66 | Variant 63 | gggtgaccgg cagcaaaatt g |
| SEQ ID NO: 67 | Variant 64 | ggctgaccgg cagcaaaatt g |
| SEQ ID NO: 68 | Variant 65 | ggttgaccgg cagcaaaatt g |
| SEQ ID NO: 69 | Variant 66 | gaaatgaccg gcagcaaaat tg |
| SEQ ID NO: 70 | Variant 67 | gaagtgaccg gcagcaaaat tg |
| SEQ ID NO: 71 | Variant 68 | gaactgaccg gcagcaaaat tg |
| SEQ ID NO: 72 | Variant 69 | gaattgaccg gcagcaaaat tg |
| SEQ ID NO: 73 | Variant 70 | gatatgaccg gcagcaaaat tg |
| SEQ ID NO: 74 | Variant 71 | gatgtgaccg gcagcaaaat tg |
| SEQ ID NO: 75 | Variant 72 | gatctgaccg gcagcaaaat tg |
| SEQ ID NO: 76 | Variant 73 | gatttgaccg gcagcaaaat tg |
| SEQ ID NO: 77 | Variant 74 | gacatgaccg gcagcaaaat tg |
| SEQ ID NO: 78 | Variant 75 | gacgtgaccg gcagcaaaat tg |
| SEQ ID NO: 79 | Variant 76 | gacctgaccg gcagcaaaat tg |
| SEQ ID NO: 80 | Variant 77 | gacttgaccg gcagcaaaat tg |
| SEQ ID NO: 81 | Variant 78 | gagatgaccg gcagcaaaat tg |
| SEQ ID NO: 82 | Variant 79 | gaggtgaccg gcagcaaaat tg |
| SEQ ID NO: 83 | Variant 80 | gagttgaccg gcagcaaaat tg |
| SEQ ID NO: 84 | Variant 81 | gtaatgaccg gcagcaaaat tg |
| SEQ ID NO: 85 | Variant 82 | gtagtgaccg gcagcaaaat tg |
| SEQ ID NO: 86 | Variant 83 | gtactgaccg gcagcaaaat tg |
| SEQ ID NO: 87 | Variant 84 | gtattgaccg gcagcaaaat tg |
| SEQ ID NO: 88 | Variant 85 | gttatgaccg gcagcaaaat tg |
| SEQ ID NO: 89 | Variant 86 | gttgtgaccg gcagcaaaat tg |
| SEQ ID NO: 90 | Variant 87 | gttctgaccg gcagcaaaat tg |
| SEQ ID NO: 91 | Variant 88 | gttttgaccg gcagcaaaat tg |
| SEQ ID NO: 92 | Variant 89 | gtcatgaccg gcagcaaaat tg |
| SEQ ID NO: 93 | Variant 90 | gtcgtgaccg gcagcaaaat tg |
| SEQ ID NO: 94 | Variant 91 | gtcctgaccg gcagcaaaat tg |
| SEQ ID NO: 95 | Variant 92 | gtcttgaccg gcagcaaaat tg |
| SEQ ID NO: 96 | Variant 93 | gtgatgaccg gcagcaaaat tg |
| SEQ ID NO: 97 | Variant 94 | gtggtgaccg gcagcaaaat tg |
| SEQ ID NO: 98 | Variant 95 | gtgctgaccg gcagcaaaat tg |
| SEQ ID NO: 99 | Variant 96 | gtgttgaccg gcagcaaaat tg |
| SEQ ID NO: 100 | Variant 97 | gcaatgaccg gcagcaaaat tg |
| SEQ ID NO: 101 | Variant 98 | gcagtgaccg gcagcaaaat tg |
| SEQ ID NO: 102 | Variant 99 | gcactgaccg gcagcaaaat tg |
| SEQ ID NO: 103 | Variant 100 | gcattgaccg gcagcaaaat tg |
| SEQ ID NO: 104 | Variant 101 | gctatgaccg gcagcaaaat tg |
| SEQ ID NO: 105 | Variant 102 | gctgtgaccg gcagcaaaat tg |
| SEQ ID NO: 106 | Variant 103 | gctctgaccg gcagcaaaat tg |
| SEQ ID NO: 107 | Variant 104 | gctttgaccg gcagcaaaat tg |
| SEQ ID NO: 108 | Variant 105 | gccatgaccg gcagcaaaat tg |
| SEQ ID NO: 109 | Variant 106 | gccgtgaccg gcagcaaaat tg |
| SEQ ID NO: 110 | Variant 107 | gccctgaccg gcagcaaaat tg |
| SEQ ID NO: 111 | Variant 108 | gccttgaccg gcagcaaaat tg |
| SEQ ID NO: 112 | Variant 109 | gcgatgaccg gcagcaaaat tg |
| SEQ ID NO: 113 | Variant 110 | gcggtgaccg gcagcaaaat tg |
| SEQ ID NO: 114 | Variant 111 | gcgctgaccg gcagcaaaat tg |
| SEQ ID NO: 115 | Variant 112 | gcgttgaccg gcagcaaaat tg |
| SEQ ID NO: 116 | Variant 113 | ggaatgaccg gcagcaaaat tg |
| SEQ ID NO: 117 | Variant 114 | ggagtgaccg gcagcaaaat tg |
| SEQ ID NO: 118 | Variant 115 | ggactgaccg gcagcaaaat tg |
| SEQ ID NO: 119 | Variant 116 | ggattgaccg gcagcaaaat tg |
| SEQ ID NO: 120 | Variant 117 | ggtatgaccg gcagcaaaat tg |
| SEQ ID NO: 121 | Variant 118 | ggtgtgaccg gcagcaaaat tg |
| SEQ ID NO: 122 | Variant 119 | ggtctgaccg gcagcaaaat tg |
| SEQ ID NO: 123 | Variant 120 | ggtttgaccg gcagcaaaat tg |
| SEQ ID NO: 124 | Variant 121 | ggcatgaccg gcagcaaaat tg |
| SEQ ID NO: 125 | Variant 122 | ggcgtgaccg gcagcaaaat tg |
| SEQ ID NO: 126 | Variant 123 | ggcctgaccg gcagcaaaat tg |
| SEQ ID NO: 127 | Variant 124 | ggcttgaccg gcagcaaaat tg |
| SEQ ID NO: 128 | Variant 125 | gggatgaccg gcagcaaaat tg |
| SEQ ID NO: 129 | Variant 126 | ggggtgaccg gcagcaaaat tg |
| SEQ ID NO: 130 | Variant 127 | gggctgaccg gcagcaaaat tg |
| SEQ ID NO: 131 | Variant 128 | gggttgaccg gcagcaaaat tg |
| SEQ ID NO: 132 | CXCR4 forward extension primer | gagctgaccg gcagcaaaat tgcaacctct acagcagtgt cctcatc |
| SEQ ID NO: 133 | CXCR4 reverse primer | ggagtgtgac agcttggaga tg |
| SEQ ID NO: 134 | Variant 129 | GAGCAGACCGGCAGCAAAATTG |
| SEQ ID NO: 135 | Variant 130 | GAGCCGACCGGCAGCAAAATTG |
| SEQ ID NO: 136 | Variant 131 | GAGCGGACCGGCAGCAAAATTG |
| SEQ ID NO: 137 | Variant 132 | GAGCTAACCGGCAGCAAAATTG |
| SEQ ID NO: 138 | Variant 133 | GAGCTCACCGGCAGCAAAATTG |
| SEQ ID NO: 139 | Variant 134 | GAGCTTACCGGCAGCAAAATTG |
| SEQ ID NO: 140 | Variant 135 | GAGCTGCCCGGCAGCAAAATTG |
| SEQ ID NO: 141 | Variant 136 | GAGCTGGCCGGCAGCAAAATTG |
| SEQ ID NO: 142 | Variant 137 | GAGCTGTCCGGCAGCAAAATTG |
| SEQ ID NO: 143 | Variant 138 | GAGCTGAACGGCAGCAAAATTG |
| SEQ ID NO: 144 | Variant 139 | GAGCTGAGCGGCAGCAAAATTG |
| SEQ ID NO: 145 | Variant 140 | GAGCTGATCGGCAGCAAAATTG |
| SEQ ID NO: 146 | Variant 141 | GAGCTGACAGGCAGCAAAATTG |
| SEQ ID NO: 147 | Variant 142 | GAGCTGACGGGCAGCAAAATTG |
| SEQ ID NO: 148 | Variant 143 | GAGCTGACTGGCAGCAAAATTG |
| SEQ ID NO: 149 | Variant 144 | GAGCTGACCAGCAGCAAAATTG |
| SEQ ID NO: 150 | Variant 145 | GAGCTGACCCGCAGCAAAATTG |
| SEQ ID NO: 151 | Variant 146 | GAGCTGACCTGCAGCAAAATTG |
| SEQ ID NO: 152 | Variant 147 | GAGCTGACCGACAGCAAAATTG |
| SEQ ID NO: 153 | Variant 148 | GAGCTGACCGCCAGCAAAATTG |
| SEQ ID NO: 154 | Variant 149 | GAGCTGACCGTCAGCAAAATTG |
| SEQ ID NO: 155 | Variant 150 | GAGCTGACCGGAAGCAAAATTG |
| SEQ ID NO: 156 | Variant 151 | GAGCTGACCGGGAGCAAAATTG |
| SEQ ID NO: 157 | Variant 152 | GAGCTGACCGGTAGCAAAATTG |
| SEQ ID NO: 158 | Variant 153 | GAGCTGACCGGCCGCAAAATTG |
| SEQ ID NO: 159 | Variant 154 | GAGCTGACCGGCGGCAAAATTG |
| SEQ ID NO: 160 | Variant 155 | GAGCTGACCGGCTGCAAAATTG |
| SEQ ID NO: 161 | Variant 156 | GAGCTGACCGGCAACAAAATTG |
| SEQ ID NO: 162 | Variant 157 | GAGCTGACCGGCACCAAAATTG |
| SEQ ID NO: 163 | Variant 158 | GAGCTGACCGGCATCAAAATTG |
| SEQ ID NO: 164 | Variant 159 | GAGCTGACCGGCAGAAAAATTG |
| SEQ ID NO: 165 | Variant 160 | GAGCTGACCGGCAGGAAAATTG |
| SEQ ID NO: 166 | Variant 161 | GAGCTGACCGGCAGTAAAATTG |
| SEQ ID NO: 167 | Variant 162 | GAGCTGACCGGCAGCCAAATTG |
| SEQ ID NO: 168 | Variant 163 | GAGCTGACCGGCAGCGAAATTG |
| SEQ ID NO: 169 | Variant 164 | GAGCTGACCGGCAGCTAAATTG |
| SEQ ID NO: 170 | Variant 165 | GAGCTGACCGGCAGCACAATTG |
| SEQ ID NO: 171 | Variant 166 | GAGCTGACCGGCAGCAGAATTG |
| SEQ ID NO: 172 | Variant 167 | GAGCTGACCGGCAGCATAATTG |
| SEQ ID NO: 173 | Variant 168 | GAGCTGACCGGCAGCAACATTG |
| SEQ ID NO: 174 | Variant 169 | GAGCTGACCGGCAGCAAGATTG |
| SEQ ID NO: 175 | Variant 170 | GAGCTGACCGGCAGCAATATTG |
| SEQ ID NO: 176 | Variant 171 | GAGCTGACCGGCAGCAAACTTG |
| SEQ ID NO: 177 | Variant 172 | GAGCTGACCGGCAGCAAAGTTG |
| SEQ ID NO: 178 | Variant 173 | GAGCTGACCGGCAGCAAATTTG |
| SEQ ID NO: 179 | Variant 174 | GAGCTGACCGGCAGCAAAAATG |
| SEQ ID NO: 180 | Variant 175 | GAGCTGACCGGCAGCAAAACTG |
| SEQ ID NO: 181 | Variant 176 | GAGCTGACCGGCAGCAAAAGTG |
| SEQ ID NO: 182 | Variant 177 | GAGCTGACCGGCAGCAAAATAG |
| SEQ ID NO: 183 | Variant 178 | GAGCTGACCGGCAGCAAAATCG |
| SEQ ID NO: 184 | Variant 179 | GAGCTGACCGGCAGCAAAATGG |
| SEQ ID NO: 185 | Variant 180 | GAGCTGACCGGCAGCAAAATTA |
| SEQ ID NO: 186 | Variant 181 | GAGCTGACCGGCAGCAAAATTC |
| SEQ ID NO: 187 | Variant 182 | GAGCTGACCGGCAGCAAAATTT |
| SEQ ID NO: 188 | Variant 183 | AGCAGACCGGCAGCAAAATTG |
| SEQ ID NO: 189 | Variant 184 | AGCCGACCGGCAGCAAAATTG |
| SEQ ID NO: 190 | Variant 185 | AGCGGACCGGCAGCAAAATTG |
| SEQ ID NO: 191 | Variant 186 | AGCTAACCGGCAGCAAAATTG |
| SEQ ID NO: 192 | Variant 187 | AGCTCACCGGCAGCAAAATTG |
| SEQ ID NO: 193 | Variant 188 | AGCTTACCGGCAGCAAAATTG |
| SEQ ID NO: 194 | Variant 189 | AGCTGCCCGGCAGCAAAATTG |
| SEQ ID NO: 195 | Variant 190 | AGCTGGCCGGCAGCAAAATTG |
| SEQ ID NO: 196 | Variant 191 | AGCTGTCCGGCAGCAAAATTG |
| SEQ ID NO: 197 | Variant 192 | AGCTGAACGGCAGCAAAATTG |
| SEQ ID NO: 198 | Variant 193 | AGCTGAGCGGCAGCAAAATTG |
| SEQ ID NO: 199 | Variant 194 | AGCTGATCGGCAGCAAAATTG |
| SEQ ID NO: 200 | Variant 195 | AGCTGACAGGCAGCAAAATTG |
| SEQ ID NO: 201 | Variant 196 | AGCTGACGGGCAGCAAAATTG |
| SEQ ID NO: 202 | Variant 197 | AGCTGACTGGCAGCAAAATTG |
| SEQ ID NO: 203 | Variant 198 | AGCTGACCAGCAGCAAAATTG |
| SEQ ID NO: 204 | Variant 199 | AGCTGACCCGCAGCAAAATTG |
| SEQ ID NO: 205 | Variant 200 | AGCTGACCTGCAGCAAAATTG |
| SEQ ID NO: 206 | Variant 201 | AGCTGACCGACAGCAAAATTG |
| SEQ ID NO: 207 | Variant 202 | AGCTGACCGCCAGCAAAATTG |
| SEQ ID NO: 208 | Variant 203 | AGCTGACCGTCAGCAAAATTG |
| SEQ ID NO: 209 | Variant 204 | AGCTGACCGGAAGCAAAATTG |
| SEQ ID NO: 210 | Variant 205 | AGCTGACCGGGAGCAAAATTG |
| SEQ ID NO: 211 | Variant 206 | AGCTGACCGGTAGCAAAATTG |
| SEQ ID NO: 212 | Variant 207 | AGCTGACCGGCCGCAAAATTG |
| SEQ ID NO: 213 | Variant 208 | AGCTGACCGGCGGCAAAATTG |
| SEQ ID NO: 214 | Variant 209 | AGCTGACCGGCTGCAAAATTG |
| SEQ ID NO: 215 | Variant 210 | AGCTGACCGGCAACAAAATTG |
| SEQ ID NO: 216 | Variant 211 | AGCTGACCGGCACCAAAATTG |
| SEQ ID NO: 217 | Variant 212 | AGCTGACCGGCATCAAAATTG |
| SEQ ID NO: 218 | Variant 213 | AGCTGACCGGCAGAAAAATTG |
| SEQ ID NO: 219 | Variant 214 | AGCTGACCGGCAGGAAAATTG |
| SEQ ID NO: 220 | Variant 215 | AGCTGACCGGCAGTAAAATTG |
| SEQ ID NO: 221 | Variant 216 | AGCTGACCGGCAGCCAAATTG |
| SEQ ID NO: 222 | Variant 217 | AGCTGACCGGCAGCGAAATTG |
| SEQ ID NO: 223 | Variant 218 | AGCTGACCGGCAGCTAAATTG |
| SEQ ID NO: 224 | Variant 219 | AGCTGACCGGCAGCACAATTG |
| SEQ ID NO: 225 | Variant 220 | AGCTGACCGGCAGCAGAATTG |
| SEQ ID NO: 226 | Variant 221 | AGCTGACCGGCAGCATAATTG |
| SEQ ID NO: 227 | Variant 222 | AGCTGACCGGCAGCAACATTG |
| SEQ ID NO: 228 | Variant 223 | AGCTGACCGGCAGCAAGATTG |
| SEQ ID NO: 229 | Variant 224 | AGCTGACCGGCAGCAATATTG |
| SEQ ID NO: 230 | Variant 225 | AGCTGACCGGCAGCAAACTTG |
| SEQ ID NO: 231 | Variant 226 | AGCTGACCGGCAGCAAAGTTG |
| SEQ ID NO: 232 | Variant 227 | AGCTGACCGGCAGCAAATTTG |
| SEQ ID NO: 233 | Variant 228 | AGCTGACCGGCAGCAAAAATG |
| SEQ ID NO: 234 | Variant 229 | AGCTGACCGGCAGCAAAACTG |
| SEQ ID NO: 235 | Variant 230 | AGCTGACCGGCAGCAAAAGTG |
| SEQ ID NO: 236 | Variant 231 | AGCTGACCGGCAGCAAAATAG |
| SEQ ID NO: 237 | Variant 232 | AGCTGACCGGCAGCAAAATCG |
| SEQ ID NO: 238 | Variant 233 | AGCTGACCGGCAGCAAAATGG |
| SEQ ID NO: 239 | Variant 234 | AGCTGACCGGCAGCAAAATTA |
| SEQ ID NO: 240 | Variant 235 | AGCTGACCGGCAGCAAAATTC |
| SEQ ID NO: 241 | Variant 236 | AGCTGACCGGCAGCAAAATTT |

### References

Steentoft, C. et al. Nat. Methods 8, 977-82 (2011) Duda, K. et al. Nucleic Acids Res. 1-16. April 21. [Epub ahead of print] (2014) Steentoft, C. et al. EMBO J. 32, 1478-88 (2013)

## Claims

1. A method for detecting an indel in a target nucleic acid derived from a targetant obtained after gene editing, said method comprising the steps of:
a) providing a gene edited targetant or DNA material from a gene edited targetant;
b) performing a triprimer amplification reaction to amplify the target nucleic acid from the gene edited targetant or the DNA material from the gene edited targetant, said reaction comprising the steps of:
i. providing a forward primer capable of annealing to a region of said target nucleic acid;
ii. providing a reverse primer capable of annealing to another region of said target nucleic acid;
wherein at least one of the forward and the reverse primers comprises a nucleic acid adaptamer sequence in its 5'-end and wherein said forward and reverse primers are flanking said target nucleic acid;
iii. providing at least one universal primer comprising a nucleic acid sequence comprising 5'-NNNTGACCGGCAGCAAAATTG-3' (SEQ ID NO: 1) or a variant thereof comprising a sequence having at least 85% identity to SEQ ID NO: 5, said universal primer being labelled by a fluorophore in its 5'-end, and said universal primer being identical to the adaptamer sequence, and said DNA material or targetant does not comprise a nucleic acid sequence identical to or complementary to the sequence of the universal primer;
thereby obtaining fluorophore-labelled amplicons;
c) analysing the size of said fluorophore-labelled amplicons in order to determine whether an indel of down to one nucleotide is present, wherein the size is detected by capillary electrophoresis or polyamide gel electrophoresis.

2. The method according to claim 1, wherein the amplification reaction is a touchdown PCR.

3. The method according to any one of the preceding claims further comprising step d) following step c) wherein the targetant is discarded if the net change in the total number of nucleic acids of the said fluorophore-labelled amplicon is equal to zero.

4. The method according to any one of the preceding claims, wherein gene editing is gene targeting.

5. The method according to anyone of the preceding claims, wherein the gene targeting is performed under the presence of a nuclease system and wherein said nuclease system is selected from a zinc finger nuclease (ZFN)-, transcription activator-like effector nuclease (TALEN)-, RNA-guided clustered regularly interspaced short palindromic repeats (CRISPR)- or Meganuclease-system targeted mutagenesis using primers comprising the desired mutations; random mutagenesis; integrative plasmids.

6. The method according to any one of the preceding claims, wherein the indel is an insertion or a deletion of at the most 10 nucleotides, such as 9 nucleotides, such as 8 nucleotides, such as 7 nucleotides, such as 6 nucleotides, such as 5 nucleotides, such as 4 nucleotides, such as 3 nucleotides, such as 2 nucleotides, such as 1 nucleotide at the most.

## Patentansprüche

1. Verfahren zum Detektieren eines Indel in einer Zielnukleinsäure, abgeleitet aus einer nach Geneditierung erhaltenen Targetante, wobei das Verfahren die folgenden Schritte umfasst:
a) Bereitstellen einer geneditierten Targetante oder von DNA-Material aus einer geneditierten Targetante;
b) Durchführen einer Triprimer-Amplifikationsreaktion zum Amplifizieren der Zielnukleinsäure aus der geneditierten Targetante oder dem DNA-Material aus der geneditierten Targetante, wobei die Reaktion die folgenden Schritte umfasst:
i. Bereitstellen eines Vorwärtsprimers mit der Fähigkeit zum Annealen an eine Region der Zielnukleinsäure;
ii. Bereitstellen eines Rückwärtsprimers mit der Fähigkeit zum Annealen an eine andere Region der Zielnukleinsäure;
wobei mindestens einer von dem Vorwärts- und dem Rückwärtsprimer eine Nukleinsäureadaptamersequenz in seinem 5'-Ende umfasst und wobei die Vorwärts- und Rückwärtsprimer die Zielnukleinsäure flankieren;
iii. Bereitstellen mindestens eines universellen Primers, umfassend eine Nukleinsäuresequenz umfassend 5'-NNNTGACCGGCAGCAAAATTG-3' (SEQ ID NO: 1) oder eine Variante davon, umfassend eine Sequenz, die mindestens 85 % Identität zu SEQ ID NO: 5 aufweist, wobei der universelle Primer in seinem 5'-Ende durch ein Fluorophor markiert ist, und der universelle Primer identisch zur Adaptamersequenz ist, und das DNA-Material oder die Targetante keine Nukleinsäuresequenz, die identisch zu oder komplementär zu der Sequenz des universellen Primers ist, umfasst;
wodurch Fluorophor-markierte Amplicons erhalten werden;
c) Analysieren der Größe der Fluorophor-markierten Amplicons, um zu bestimmen, ob ein Indel von bis zumindest einem Nukleotid vorliegt, wobei die Größe mittels Kapillarelektrophorese oder Polyamidgelelektrophorese detektiert wird.

2. Verfahren nach Anspruch 1, wobei es sich bei der Amplifikationsreaktion um eine Touchdown-PCR handelt.

3. Verfahren nach einem der vorhergehenden Ansprüche, das ferner im Anschluss an Schritt c) den Schritt d) umfasst, in dem die Targetante verworfen wird, wenn die Netto-Änderung der Gesamtzahl von Nukleinsäuren des Fluorophor-markierten Amplicons gleich null ist.

4. Verfahren nach einem der vorhergehenden Ansprüche, wobei es sich bei der Geneditierung um ein Gen-Targeting handelt.

5. Verfahren nach einem der vorhergehenden Ansprüche, wobei das Gen-Targeting durchgeführt wird in Gegenwart eines Nukleasesystems, und wobei das Nukleasesystem ausgewählt ist aus einer Zinkfingernuklease (ZFN)-, Transkriptionsaktivator-ähnlichen-Effektornuklease (TALEN)-, RNA-geleiteten Clustered-Regularly Interspaced-Short-Palindromic-Repeats (CRISPR)- oder Meganuklease-System-zielgelenkten Mutagenese unter Verwendung von die gewünschten Mutationen umfassenden Primern; Zufallsmutagenese; integrativen Plasmiden.

6. Verfahren nach einem der vorhergehenden Ansprüche, wobei es sich bei dem Indel um eine Insertion oder eine Deletion von höchstens 10 Nukleotiden, wie im Höchstfall 9 Nukleotiden, wie 8 Nukleotiden, wie 7 Nukleotiden, wie 6 Nukleotiden, wie 5 Nukleotiden, wie 4 Nukleotiden, wie 3 Nukleotiden, wie 2 Nukleotiden, wie 1 Nukleotid, handelt.

## Revendications

1. Procédé de détection d'un indel dans un acide nucléique cible dérivé d'un agent de ciblage obtenu après édition génique, ledit procédé comprenant les étapes de :
a) fourniture d'un agent de ciblage modifié par un gène ou d'un matériau d'ADN provenant d'un agent de ciblage modifié par un gène ;
b) réalisation d'une réaction d'amplification par triamorce pour amplifier l'acide nucléique cible provenant de l'agent de ciblage génétiquement modifié ou du matériau d'ADN provenant de l'agent de ciblage génétiquement modifié, ladite réaction comprenant les étapes de :
i. fourniture d'une amorce sens pouvant s'hybrider à une région dudit acide nucléique cible ;
ii. fourniture d'une amorce antisens pouvant s'hybrider à une autre région dudit acide nucléique cible ;
dans lequel au moins l'une des amorces sens et antisens comprend une séquence d'adaptamère d'acide nucléique à son extrémité 5' et dans lequel lesdites amorces sens et antisens flanquent ledit acide nucléique cible ;
iii. fourniture d'au moins une amorce universelle comprenant une séquence d'acide nucléique comprenant 5'-NNNTGACCGGCAGCAAAATTG-3' (SEQ ID NO : 1) ou un variant de celui-ci comprenant une séquence ayant au moins 85 % d'identité avec SEQ ID NO : 5, ladite amorce universelle étant marquée par un fluorophore à son extrémité 5', et ladite amorce universelle étant identique à la séquence d'adaptamère, et ledit matériau d'ADN ou agent de ciblage ne comprend pas une séquence d'acide nucléique identique ou complémentaire de la séquence de l'amorce universelle ;
obtenant ainsi des amplicons marqués par un fluorophore ;
c) analyse de la taille desdits amplicons marqués par fluorophore afin de déterminer si un indel de jusqu'à un seul nucléotide est présent, la taille étant détectée par électrophorèse capillaire ou électrophorèse sur gel de polyamide.

2. Procédé selon la revendication 1, dans lequel la réaction d'amplification est une PCR touchdown.

3. Procédé selon l'une quelconque des revendications précédentes comprenant en outre l'étape d) suivant l'étape c) dans lequel l'agent de ciblage est rejeté si le changement net du nombre total d'acides nucléiques dudit amplicon marqué par un fluorophore est égal à zéro.

4. Procédé selon l'une quelconque des revendications précédentes, dans lequel l'édition génique est un ciblage génique.

5. Procédé selon l'une quelconque des revendications précédentes, dans lequel le ciblage génique est effectué en présence d'un système de nucléase et dans lequel ledit système de nucléase est choisi parmi une nucléase à doigt de zinc (ZFN), une nucléase effectrice de type activateur de transcription (TALEN), des répétitions palindromiques courtes groupées régulièrement espacées guidées par ARN (CRISPR) ou une mutagenèse ciblée par un système de méganucléase utilisant des amorces comprenant les mutations souhaitées ; une mutagenèse aléatoire ; des plasmides intégratifs.

6. Procédé selon l'une quelconque des revendications précédentes, dans lequel l'indel est une insertion ou une délétion d'au plus 10 nucléotides, tels que 9 nucléotides, tels que 8 nucléotides, tels que 7 nucléotides, tels que 6 nucléotides, tels que 5 nucléotides, tels que 4 nucléotides, tels que 3 nucléotides, tels que 2 nucléotides, tels que 1 nucléotide au plus.
